(19) **Europäisches Patentamt / European Patent Office / Office européen des brevets**

(11) **EP 0 755 407 B1**

(12) **EUROPEAN PATENT SPECIFICATION**

(45) Date of publication and mention
of the grant of the patent:
**11.04.2007 Bulletin 2007/15**

(51) Int Cl.:
**C07K 14/435** (2006.01)   **A61K 38/17** (2006.01)

(21) Application number: **95916952.5**

(86) International application number:
**PCT/US1995/004481**

(22) Date of filing: **12.04.1995**

(87) International publication number:
**WO 1995/028424 (26.10.1995 Gazette 1995/46)**

(54) **PHARMACEUTICAL PEPTIDE FORMULATIONS FOR TREATMENT OF DUST MITE ALLERGY**

PHARMAZEUTISCHE PEPTIDE FORMULIERUNGEN ZUR BEHANDLUNG VON STAUBMILBEN ALLERGIE

FORMULATIONS PHARMACEUTIQUES A BASE DE PEPTIDES UTILES POUR TRAITER L'ALLERGIE AUX ACARIENS DETRITICOLES

(84) Designated Contracting States:
**AT BE CH DE DK ES FR GB GR IE IT LI LU MC NL PT SE**

(30) Priority: **14.04.1994 US 227772**

(43) Date of publication of application:
**29.01.1997 Bulletin 1997/05**

(73) Proprietor: **Merck Patent GmbH**
**64293 Darmstadt (DE)**

(72) Inventors:
• **CHEN, Xian**
  **North Chelmsford, MA 01863 (US)**
• **EVANS, Sean**
  **Acton, MA 01720 (US)**
• **SHAKED, Ze'ev**
  **Berkely, CA 94707 (US)**
• **FRANZEN, Henry, M.**
  **Watertown, MA 02172 (US)**
• **KUO, Mei-chang**
  **Winchester, MA 01890 (US)**

(56) References cited:
**WO-A-92/04445**      **WO-A-93/08279**
**WO-A-94/24281**

• **DATABASE WPI Section Ch, Week 9415 Derwent Publications Ltd., London, GB; Class B04, AN 94-126807 & ZA,A,9 302 677 ( IMMULOGIC PHARM CORP) , 26 January 1994**
• **MOLECUALR IMMUNOLOGY, vol. 29, no. 6, June 1992 OXFORD (GB), pages 739-749, JEANNIN P. ET AL. 'Specific histamine release capacity of peptides selected from the modified Der P I protein ...'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

Background of the Invention

Approximately 500 million people around the world are allergic to dust mites and it is believed that allergy to dust mites may be a factor in 50% to 80% of patients with asthma as well as a numerous cases of eczema, hay fever, and other allergic symptoms. Dust mites are ubiquitous in homes, and thrive in beds, draperies and carpets. It is extremely difficult to remove them from the living environment and an allergic individual is constantly exposed to the allergen and such constant exposure may cause the allergic condition to worsen with time. Thus, allergy to dust mite may be chronic and difficult to treat.

Allergy to mites of the genus *Dermatophagoides* has been associated with a number of allergic conditions such as asthma, rhinitis, and ectopic dermatitis. Two species, *D. pteronyssinus* and *D. farinae* predominate and as a result, considerable effort has been expended in trying to identify allergens produced by these two species. Researchers have documented the importance of responses to the Group I (e.g. *Der p I,* and *Der f* I)and Group II (e.g. *Der p* II and *Der f* II) protein allergens. For example, it has been documented that over 60% of patients have at least 50% of their anti-mite antibodies directed toward these proteins(e.g., Lind, P. et al., Allergy, 39:259-274 (1984); van der Zee, J.S. et al., Journal Allergy and Clinical Immunology, 81:884-896 (1988)). It is possible that children show a greater degree of reactivity to the Group I and Group II allergens (Thompson, P.J., et al., Immunology, 64:301-314 (1988)).

A concerted effort has been made to characterize by gene cloning the major allergens from both *D. pteronyssinus* and *D. farinae.* Consequently, several publications have reported the complete nucleotide sequences of several allergens including *Der p* I (Thomas, W.R., et al., International Archives of Allergy and Applied Immunology. **85**:127-129 (1988); and Chua, K.Y., et al., Journal of Experimental Medicine, **167**:175-182 (1988)), *Der p* II (Chua, K.Y., et al., International Archives of Allergy and Applied Immunology, **91**:118-123 (1990)), *Derf* I (Dilworth, R.J., et al., Clinical and Experimental Allergy, **21**:25-32 (1891)), *Der f* II (Yuuki, T., et al., Japan Journal Allergol., **39**:557-461 (1990); and Trudinger, M., et al., Clinical and Experimental Allergy, **21**:33-37 (1991)) and a low molecular weight allergen (Ovey, E.R., et al., Journal of Experimental Medicine, **170**:1457-1462 (1989)).

The published nucleotide sequences of cDNAs encoding *Der p* I and *Der f*I demonstrate that these two proteins are highly homologous at the amino acid level (81 % identity) and that the mature protein products are comprised of 222 and 223 residues, respectively (Chua, K.Y., et al., Journal of Experimental Medicine, **167**:175-182 (1988); and Dilworth, R.J., et al., supra)). The protein allergens *Der p* II and *Der f* II are both comprised of 129 residues, and are also highly homologous (88% identity) in amino acid sequence (Trudinger, M., et al. supra; Yuuki, T., et al. supra); Chua, K.Y., et al, International Archives of Allergy and Applied Immunology, **91**:118-123 (1990)).

The isolation of cDNAs clones encoding *Der p* I and *Der p* II has permitted antibody binding studies on the recombinant antigens (Green, W.K., et al., International Archives of Allergy and Applied Immunology. **92**:30-38 (1990); Chua, K.Y., et al., International Archives of Allergy and Applied Immunology, 91:124-129 (1990)). Complementary DNA fragments of *Der p* I have been expressed in E. coli and IgE binding studies with pooled human mite allergic IgE sera have demonstrated binding and non-binding regions throughout the molecule (Thomas, W.R., et al., In: Epitopes of Atopic Allergens. Proceedings of Workshop from XIV Congress of the European Academy of Allergy and Clinical Immunology, Berlin, Sept. 1989. pp 77-82). T cell epitopes of *Der p* I have been reported (O'Hehir, R.E., et al., Annual Review Immunology, **9**:67-95 (1991); Stewart, G.A., et al., In: Epitopes of Atopic Allergens. Proceedings of Workshop from XIV Congress of the European Academy of Allergy and Clinical Immunology. Berlin, Sept. 1989. pp 41-47; Yessel, H., et al., In: T cell Activation in Health and Disease: Discrimination Between Immunity and Tolerance, Conference 22-26 Sept., 1990, Trinity College, Oxford, U.K. and Hessel, H., et al., Journal of Immunology, **148**(3): 738-745 (Feb. 1, 1992).

Presently, desensitization therapy is used for the treatment of house dust mite allergy. Such therapy includes administration of an extract derived from whole house dust mite cultures to an allergic individual. Desensitization therapy using such extract has drawbacks in that it can elicit anaphylaxis if high doses are used, whereas when low doses are used to avoid anaphylaxis, treatment is not highly effective and must be continued for several years to build up tolerance to the extract.

WO93/08279 and WO94/24281 disclose improved compositions and methods for treatment of allergy to house dust mite which greatly minimizes the potential adverse effects associated with conventional desensitization therapy using whole house dust mite extract. WO93/08279 and WO94/24281 disclose isolated antigenic fragments or peptide derived from *Der p* I, *Der p* II, *Der f* I and *Der f* II which when administered to a dust mite sensitive individual, are capable of down regulating the allergic response of the individual to house dust mite. Such down regulation of the allergic response of the individual results in diminution or alleviation of the classic symptoms of allergy including asthmatic symptoms induced by house dust mite. Such antigenic fragments are disclosed in WO93/08279 and WO94/24281 as being capable of eliciting a T cell response such as stimulation (i.e. T cell proliferation or lymphokine secretion) and/or are capable of inducing T cell nonresponsiveness or reduced T cell responsiveness when challenged with a house dust mite allergen. In addition, WO93/08279 and WO94/24281 disclose that the most preferred peptides derived from *Der p* and *Der f*

Group I and Group II protein allergens suitable for therapeutic use do not bind IgE specific for such *Der p* and *Der f* proteins, or bind IgE to a substantially lesser extent than the native dust mite protein allergen, thereby reducing or eliminating the possibility of anaphylaxis in a treatment regimen which includes such peptides. Furthermore, WO93/08279 and WO94/24281 disclose that given the cross reactivity within the interspecies Group I allergens (i.e., *Der p* I and *Der f* I) and the interspecies Group II allergens (i.e., *Der p* II and *Der f* II), peptides of Group I, for example, derived from one species (e.g., *Der p* I) are capable of eliciting a T cell response from T cells specific for the other species (e.g., *Der f* I), and vice-versa. Finally, WO93/08279 and WO94/24281 disclose peptides which possess the characteristics described above.

As a result of extensive preformulation efforts, the present invention provides novel compositions and multipeptide formulations of peptides derived from *Der p* and *Der f Group* I and Group II protein allergens which are optimal for preparation of a drug product suitable for use in treating house dust mite allergy in humans and other mammals. Such novel compositions and multipeptide formulations of *Der p* and *Der f* protein allergen peptides for use as an optimized human drug product have not previously been disclosed or contemplated.

Summary of the Invention

The present invention provides novel therapeutic compositions and multipeptide formulations of peptides and modified peptides of Group I and Group II *Der p* and *Der f* derived protein allergens. Such novel therapeutic compositions and multipeptide formulations are the result of a preformulation scheme to develop an optimized drug product for therapeutic treatment of humans suffering from allergy to house dust mite allergens. The *Der p* and *Der f* peptides and modified peptides in acordance with the invention possess certain unique characteristics which render them particularly suitable for drug product formulation. Therapeutic compositions and multipeptide formulations of the invention have been optimized to accommodate and maintain the unique characteristics of the peptides and modified peptides derived from house dust mite protein allergens and at the same time provide maximum therapeutic effect when used in therapeutic regimens for the treatment of house dust mite allergy in humans. The invention further provides novel modified peptides derived from *Der p* I and *Der p* II protein allergens.

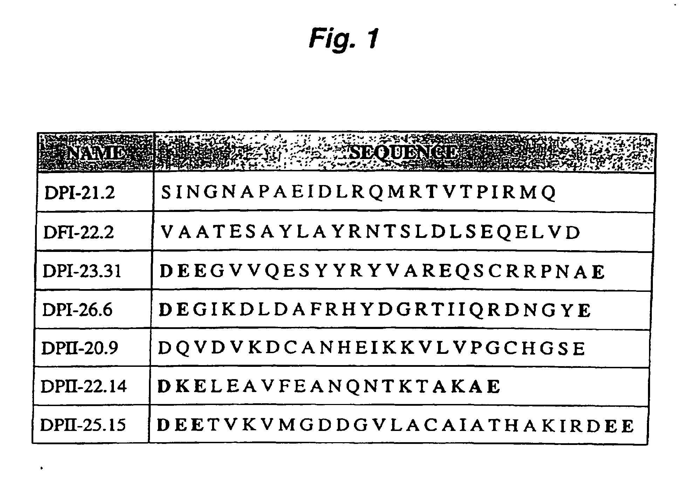

Accordingly, the invention provides a therapeutic composition comprising at least one isolated peptide selected from the group consisting of: DPI-21.2 (SEQ ID NO:27), and DFI-22.2 (SEQ ID NO:28) and at least one peptide selected from the group consisting of DPI-23.31 (SEQ ID NO:29), DPI-26.6 (SEQ ID NO:30), DPII-20.9 (SEQ ID NO:31), DPII-22.14 (SEQ ID NO:32) and DPII-25.15 (SEQ ID NO:33) all as shown in Figure 1. The composition is preferably for use in therapy.

In another aspect, the invention provides a therapeutic composition comprising: a) peptides DFI-22.2 (SEQ ID NO:28); DPI 23.1 (SEQ ID NO:29) and DPII-22.14 (SEQ ID NO:32); b) peptides DPI-21.2 (SEQ ID NO:27), DPI-26.6 (SEQ ID NO:30), and DPII-25.15 (SEQ ID NO:33); c) peptides DPI-21.2 (SEQ ID NO:27), DFI-22.22 (SEQ ID NO:28), DPI-23.31 (SEQ ID NO:29), DPI-26.6 (SEQ ID NO:30), DPII-22.14 (SEQ ID NO:32) and DPII-25.15 (SEQ ID NO:33); or d) peptides DPI-21.2 (SEQ ID NO:27), DFI-22.22 (SEQ ID NO:28), DPI-23.31 (SEQ ID NO:29), DPI-26.6 (SEQ ID NO:30), DPII-20.9 (SEQ ID NO:31), DPII-22.14 (SEQ ID NO:32) and DPII-25.1 5 (SEQ ID NO:33). The composition is preferably for use in therapy.

In another aspect, the invention provides a multipeptide formulation for pharmaceutical administration comprising: a) at least one isolated peptide selected from the group consisting of DPI-21.2 (SEQ ID NO:27), and DFI-22.2 (SEQ ID NO:28) and at least one peptide selected from the group consisting of DPI-23.31 (SEQ ID NO:29), DPI-26.6 (SEQ ID NO:30), DPII-20.9 (SEQ ID NO:31), DPII-22.14 (SEQ ID NO:32) and DPII-25.15 (SEQ ID NO:33), each peptide being soluble and stable at a pH in the range of pH 6.0 to pH 8.0 and wherein said formulation comprises a sufficient percentage of T cell activity of Group I and Group II *Der f* and *Der p* house dust mite allergens; b) a pharmaceutically acceptable carrier; and optionally c) one or more of EDTA and a pharmaceutically acceptable counter ion. Preferably the formulation comprises at least 37% of T cell activity of Group I and Group II *Der f* and *Der p* house dust mite allergens.

In another aspect, the invention provides an optimised multipeptide formulation suitable for therapeutic treatment of humans suffering from allergy to house dust mite comprising:

a) peptides DPI-21.2 (SEQ ID NO:27); DFI-22.22 (SEQ ID NO:28), DPI-23.31 (SEQ ID NO:29), DPI-26.6 (SEQ ID NO:30), DPII-22.14 (SEQ ID NO:32) and DPII-25.15 (SEQ ID NO:33); and optionally DPII-20.9; each peptide being in a concentration of 0.75mg per peptide; 0.05M sodium phosphate U.S.P.; 5% w/v mannitol U.S.P.; 0.1 mg/ml EDTA disodium dihydrate U.S.P.; and sterile water for injection U.S.P.; wherein said formulation has a final pH of 7.2-7.4; or b) peptides DFI-22.2 (SEQ ID NO:28); DPI-23.1 (SEQ ID NO:29) and DPII-22.14 (SEQ ID NO:32); each peptide being in a concentration of 0.75 mg per peptide; 0.05M sodium phosphate U.S.P.; 5% w/v mannitol U.S.P.; 0.1 mg/ml EDTA disodium dihydrate U.S.P.; and sterile water for injection U.S.P.; wherein said formulation has a final pH of 7.0 ; or c) peptides DP1-21.2 (SEQ ID NO:27), DPI-26.6 (SEQ ID NO:30), and DPII-25.15 (SEQ ID NO:33); each peptide being in a concentration of 0.75 mg per peptide; 0.05M sodium phosphate U.S.P.; 5% w/v mannitol U.S.P.; 0.1 mg/ml EDTA disodium dihydrate U.S.P.; and sterile water for injection U.S.P.; wherein said formulation has a final pH of 6.2. Preferably, the formulation is for use in therapy.

In another aspect, the invention provides first and second optimised multipeptide formulations for simultaneous or sequential use in therapy, wherein said first formulation comprises peptides DFI-22.2 (SEQ ID NO:28); DPI-23.1 (SEQ ID NO:29) and DPII-22.14 (SEQ ID NO:32); each peptide being in a concentration of 0.75 mg per peptide; 0.05M sodium phosphate U.S.P.; 5% w/v mannitol U.S.P.; 0.1 mg/ml EDTA disodium dihydrate U.S.P.; and sterile water for injection U.S.P.; having a final pH of 7.0; and wherein said second optimised multipeptide formulation comprises peptides DPI-21.2 (SEQ ID NO: 27), DPI-26.6 (SEQ ID NO:30), and DPII-25.15 (SEQ ID NO:33); each peptide being in a concentration of 0.75 mg per peptide; 0.05M sodium phosphate U.S.P.; 5% w/v mannitol U.S.P.; 0.1 mg/ml EDTA disodium dihydrate U.S.P.; and sterile water for injection U.S.P.; having a final pH of 6.2. Preferably the formulation is for use in treating sensitivity to house dust mite allergen.

Description of the Drawings

Fig. 1 shows the amino acid sequences of the "unique" peptides in accordance with the invention including the novel "unique" peptides of the invention.

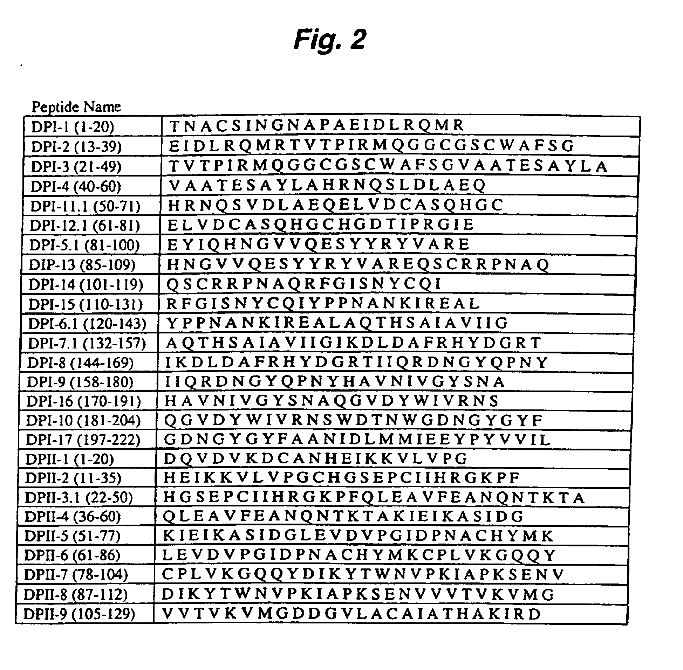

Fig. 2 shows overlapping peptides derived *Der p* I and *Der p* II protein allergens used in T cell studies described herein.

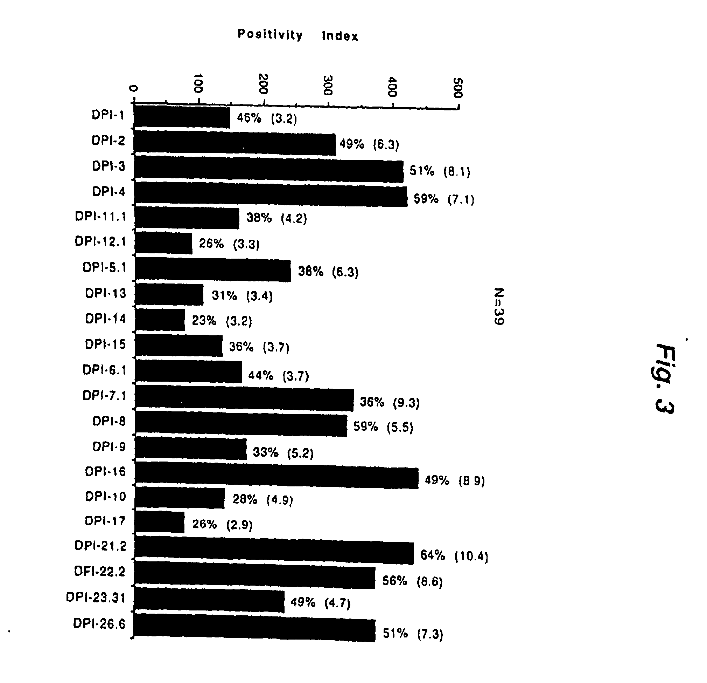

Fig. 3 is a graphic representation depicting T cell responses to the overlapping *Der p* I - peptides shown in Fig. 2 and the Group I "unique" peptides, DPI-21.2 (SEQ. ID. NO. 27), DFI-22.2 (SEQ. ID. NO. 28), DPI-23.31 (SEQ. ID. NO. 29), DPI-26.6 (SEQ. ID. NO. 30), shown in Fig. 1. The mean S.I. shown above each bar (in parenthesis) as well as the percentage of responses, the positivity index (mean S.I. multiplied by percentage of responses), is the Y axis.

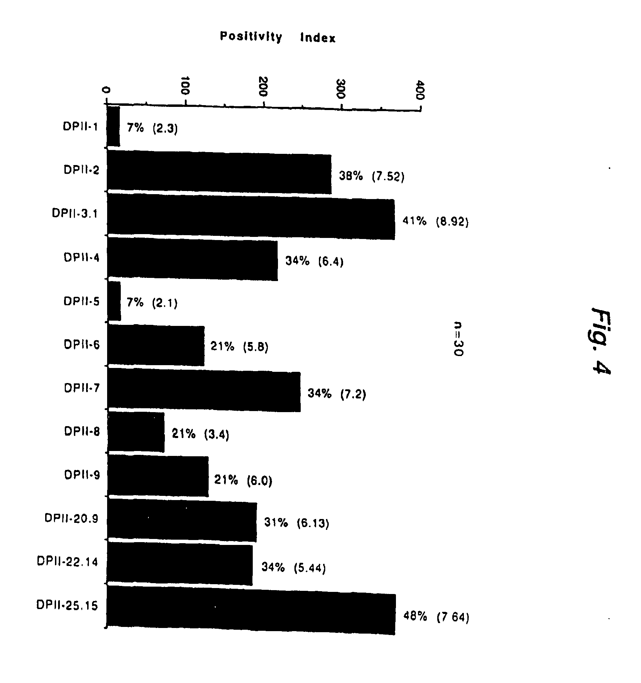

Fig. 4 is a graphic representation depicting T cell responses to the overlapping *Der p* II peptides, DPII-20.9 (SEQ. ID. NO. 31), DPII-22.14 (SEQ. ID. NO: 32), and DPII-25.15 (SEQ. ID. NO. 33), shown in Fig. 2 and the *Der p* II "unique" peptides shown in Fig. 2. The mean S.I. shown above each bar (in parenthesis) as well as the percentage of responses, the positivity index (mean S.I. multiplied by percentage of responses), is the Y axis.

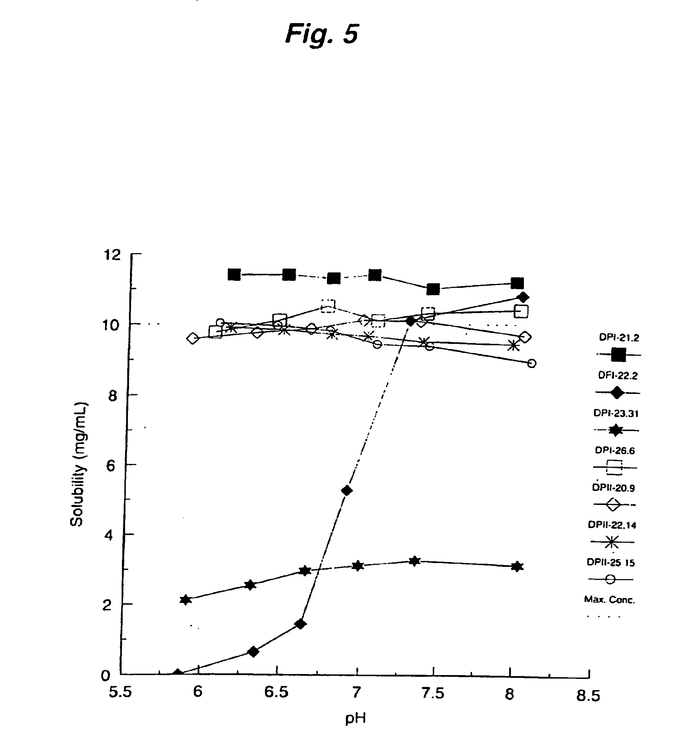

Fig. 5 is a pH-solubility profile of "unique" candidate peptides DPI-21.2 (SEQ. ID. NO. 27), DFI-22.2 (SEQ. ID. NO. 28), DPI-23.31 (SEQ. ID. NO. 29), DPI-26.6 (SEQ. ID. NO. 30), DPII-20.9 (SEQ. ID. NO. 31), DPII-22.14 (SEQ. ID. NO. 32) and DPII-25.15 (SEQ. ID. NO. 33), in 50mM sodium phosphate buffers with 5% mannitol. Solubility is measured in mg/ml (y axis) over a pH range of pH 5.5 to pH 8.5 at about 22°C $\pm$ 2.

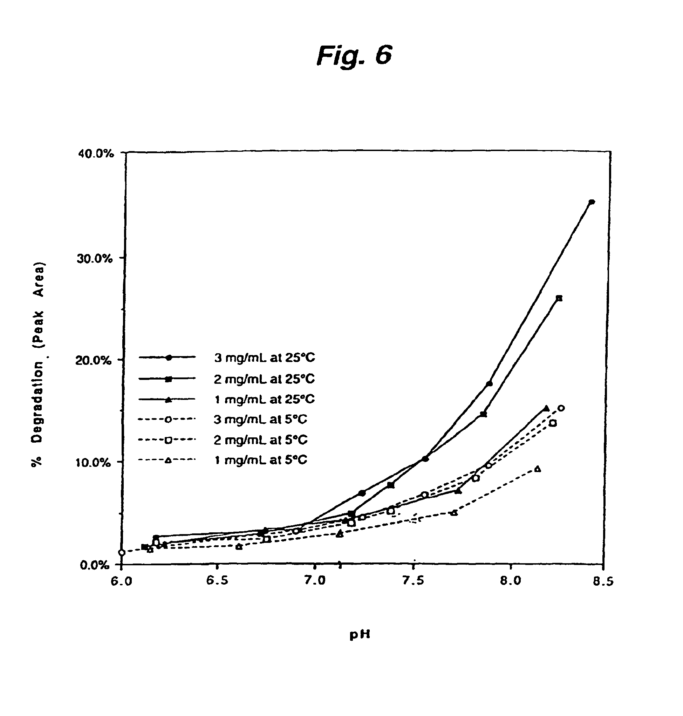

Fig. 6 is a pH-stability profile of candidate peptides DPI-21.2 (SEQ. ID. NO. 27), DFI-22.2 (SEQ. ID. NO. 28), DPI-23.31 (SEQ. ID. NO. 29), DPI-26.6 (SEQ. ID. NO. 30), DPII-20.9 (SEQ. ID. NO. 31), DPII-22.14.(SEQ. ID. NO. 32) and DPII-25.15 (SEQ. ID. NO. 33) in an equal concentration combination. Degradation of peptide is calculated as % degradation (determined by peak area using HPLC analysis) of peptide observed after 24 hours at about 22°C $\pm$ 2 and about 5°C, at various theoretical concentrations of 3.0, 2.0 and 1.0 mg/ml of peptide over a pH range of 6.0 to 8.5.

Detailed Description of the Invention

The patent and scientific literature referred to herein establishes the knowledge that is available to those with skill in the art.

The present invention relates to novel modified *Der p* I. and *Der p* II peptides which are in combination with other *Der p* I and *Der f* I peptides are a part of a preformulation scheme to develop an optimized drug product for therapeutic treatment of humans suffering from allergy to house dust mite allergen. Such peptides and modified peptides possess certain unique characteristics which render them particularly suitable for drug product formulation, and may be referred to herein as "unique" peptides.

In accordance with pharmaceutical chemistry, preformulation is the process of optimizing a drug through determination and/or definition of those physical and chemical properties considered important in the formulation of a stable, effective, and safe dosage form. The possible interactions with the various components intended for use in the final drug product are also considered. Preformulation is an intensive effort that includes the study of such parameters as solubility, pH profile of stability, and drug-excipient interactions, which may have a profound effect on a drug's physiological availability and physical and chemical stability. The data obtained from such studies are integrated with those obtained from preliminary pharmacological and biochemical studies of the active drug component thus providing information that permits the selection of the best drug form, and the most desirable excipients for use in its development.

The development of an optimum formulation of active drug component and excipients is complex and many factors influence formulation properties. The high degree of uniformity, the physiological availability and the therapeutic quality expected of pharmaceuticals can only be achieved by considerable effort and expertise. Flexibility is also an important factor in preformulation. Numerous excipients, stabilizers counter ions and the like may have to be tested in order to find those compatible with the active drug component of the formulation. Multiple modifications of the active component may become necessary to successfully formulate a drug product. Such modifications must not effect the overall therapeutic effectiveness of the drug but at the same time, must render the drug more suitable for formulation.

As a part of a preformulation scheme to provide an optimized drug product suitable for use in humans and other mammals for treating sensitivity to house dust mite, it was determined that the active component (referred to herein as a "peptide" or "candidate peptide" or "unique peptide") in such formulation should possess the following characteristics which would render such peptides "unique" among all of the possible peptides derived from the *Der p* I, *Der p* II and *Der f* I protein allergen sequences. First, a unique peptide should alone or in combination with other unique peptides comprise a sufficient percentage of the T cell reactivity of the *Der p* and *Der f* protein allergens to induce T cell nonresponsiveness or reduced T cell responsiveness in a substantial percentage of the individuals sensitive to house dust mite allergen. Second, the candidate peptide should possess the characteristic of "superior solubility" which is defined herein as solubility of greater than 3 mg/ml at a pH in a pH. range of pH 6 to pH 8 in an aqueous buffer. Third, the peptide is stable in an aqueous buffer at a pH in a pH range from pH 6 to pH 8. Candidate peptides derived from *Der p* and *Der f* protein allergens which have been determined to be "unique" peptides of use in the invention are DPI-21.2 (SEQ. ID. NO. 27), DFI-22.2 (SEQ. ID. NO. 28), DPI-23.31 (SEQ. ID. NO. 29), DPI-26.6 (SEQ. ID. NO. 30), DPII-20.9 (SEQ. ID. NO. 3 1), DPII-22.14 (SEQ. ID. NO. 32) and DPII-25.15 (SEQ. ID. NO. 33) all as shown in Fig. I.

In accordance with the first characteristic, Those peptides found to elicit a T cell response such as T cell proliferation or lymphokine secretion (i.e. comprise at least one T cell epitope), or induce T cell non-responsiveness or reduced T cell responsiveness are understood to have T cell reactivity. T cell epitopes are believed to be involved in initiation and perpetuation of the immune response to a protein allergen which is responsible for the clinical symptoms of allergy. These T cell epitopes are thought to trigger early events at the level of the T helper cell by binding to an appropriate HLA molecule on the surface of an antigen presenting cell and stimulating the relevant T cell subpopulation. These events lead to T cell proliferation. lymphokine secretion, local inflammatory reactions, recruitment of additional immune cells to the site, and activation of the B cell cascade leading to production of antibodies. One isotype of these antibodies, IgE, is fundamentally important to the development of allergic symptoms and its production is influenced early in the cascade of events, at the level of the T helper cell, by the nature of the lymphokines secreted. A T cell epitope is the basic element or smallest unit of recognition by a T cell receptor, where the epitope comprises amino acids essential to receptor recognition. It is believed that exposure of house dust mite allergic patients to isolated house dust mite Group I and Group II protein allergen peptides which comprise at least one T cell epitope may cause T cell non-responsiveness of appropriate T cell subpopulations such that they become unresponsive or have reduced responsiveness to the protein allergen and do not participate in stimulating an immune response upon such exposure for example, via anergy, tolerance, or apoptosis, the ability to modify the lymphokine secretion profile as compared with exposure to the naturally occurring autoantigen; and /or the ability to cause induction of T suppresser cells.

To determine peptides having T cell reactivity and comprising at least one T cell epitope, isolated peptides are tested by, for example, T cell biology techniques, to determine whether the peptides elicit a T cell response or induce T cell non-responsiveness. As discussed in the Examples human T cell stimulating activity can be tested by culturing T cells obtained from an individual sensitive to house dust mite allergen, (i.e., an individual who has an IgE mediated immune response to house dust mite allergen) with a peptide or modified peptide derived from a *Der p* or *Der f* Group I or Group II protein allergen and determining whether proliferation of T cells occurs in response to the peptide as measured, e.g., by cellular uptake of tritiated thymidine. Stimulation indices for responses by T cells to peptides can be calculated as the maximum counts per minute (CPM) in response to a peptide divided by the control CPM. A stimulation index (S.I.) equal to or greater than two times the background level is considered "positive". Positive results are used to calculate the mean stimulation index for each peptide for the group of patients tested. Peptides suitable as candidates for formulation into a final drug product have a mean T cell stimulation index of greater than or equal to 2.0 and preferably higher, (e.g. at least 2.5, more preferably at least 3.5, more preferably at least 4.0, more preferably at least 5, even more preferably at least 7 and most preferably at least about 9).

For therapeutic purposes, candidate peptides are recognized by at least 10%, more preferably at least 20%, more preferably at least 30% and even more preferably at least 40% or more of individuals in a population of individuals sensitive to house dust mite allergen. In addition, preferred candidate peptides have a positivity index (P.I.) of at least about 100, more preferably at least about 250 and most preferably at least about 350. The positivity index for a peptide is determined by multiplying the mean T cell stimulation index by the percent of individuals, in a population of individuals sensitive to house dust mite allergen (e.g., preferably at least 15 individuals, more preferably at least 30 individuals or more), who have a T cell stimulation index to such peptide of at least 2.0. Thus, the positivity index represents both the strength of a T cell response to a peptide (S.I.) and the frequency of a T cell response to a peptide in a population of individuals sensitive to house dust mite allergen.

To determine whether a peptide (candidate peptide) or a combination of candidate peptides are likely to comprise a sufficient percentage of the T cell reactivity of house dust mite protein allergens, to induce T cell nonresponsiveness in a substantial percentage of a population of individuals sensitive to house dust mite allergen, an algorithm can be used. In accordance with one such algorithm, a set of overlapping peptides is produced by systematically dividing the protein allergen(s) of interest into at least two overlapping peptide regions of desired lengths (e.g., of about 12-30 amino acid residues in length, preferably not longer than about 25 amino acid residues in length with about 5-15 amino acid residues of overlap). This division into peptide regions can be arbitrary, can be made according to an algorithm, or can be wholly or partially based on regions of house dust mite Group I and/or Group II protein allergens known to comprise at least one T cell epitope. Preferably, at least 50% of the entire house dust mite protein allergen sequence and more preferably, the entire house dust mite protein allergen sequence is divided into two or more peptides. A human T cell stimulation index is determined for each of the peptides in an *in vitro* T cell proliferation assay as described herein for each individual tested in a population of individuals sensitive to the protein antigen. For example both WO93/08279 and WO94/24281 disclose T cell studies with overlapping peptides derived from *Der p* I, *Der p* II, *Der f* I and *Der f* II. A candidate peptide or combination of candidate peptides is selected based, at least in part, on the mean human T cell stimulation index of the candidate peptide in the set of peptides tested and the positivity index of the candidate peptide in the set of peptides tested (see, Figs. 3 and 4). The human T cell stimulation index for the candidate peptide(s) is summed. For each individual, the human T cell stimulation index for the candidate peptide(s) is divided by the sum of the human T cells stimulation indices of the remaining peptides in the set of peptides tested to determine a percent of T cell reactivity as shown below:

$$(1) \quad \text{\% T Cell Reactivity of a candidate peptide(s)} = \frac{\text{Candidate S.I.}}{\text{Sum of S.I. of the set of Overlapping peptides}} \times 100$$

Alternatively, the presence of T cell epitopes in the candidate peptide dependent on amino acids residues in an overlapping peptide located at either the N-terminus or C-terminus of the candidate peptide in the amino acid sequence of the protein antigen, but which epitopes are not present in the candidate peptide can be considered in calculating the percent of T cell reactivity in the candidate peptide by use of the following formula:

$$(2) \quad \text{\% T Cell Reactivity of a candidate peptide(s)} =$$

$$\frac{N_T \text{ flanking peptide S.I. + Candidate peptide S.I. + } C_T \text{ flanking peptide S.I}}{\text{Sum of S.I. of the set of overlapping peptides}} \times 100$$

In this formula, "$N_T$ flanking peptide" refers to a peptide which comprises amino acid residues which overlap with amino acid residues located at the N-terminus of the candidate peptide in the amino acid sequence of the protein antigen from which the peptide is derived; "$C_T$ flanking peptide" refers to a peptide which comprises amino acid residues which overlap with amino acid residues located a the C-terminus of the candidate peptide in the amino acid sequence of the protein antigen from which the peptide is derived. In this calculation stimulation indices for the candidate peptide, the N-terminal flanking peptide and the C-terminal flanking peptide are added and divided by the sum total of the stimulation indices for the entire set of overlapping peptides obtain a percent of T cell reactivity for the candidate peptide. If a combination of two or more candidate peptides is selected each of which contains amino acid residues which overlap, this calculation cannot be used to determine a percent of T cell reactivity for each candidate peptide separately. However, a total percent of T cell reactivity for the combination of candidate peptides can be obtained. In this situation, the stimulation indices for all of the candidate peptides which overlap is included in the calculation.

The values obtained for the percentage of T cell reactivity for the candidate peptide or combination of peptides in each individual tested can be expressed as a range of the lower and higher values of the results of the above described calculations. By either of the above calculations, the percent is obtained for at least about twenty (20) and preferably at least about thirty (30) individuals sensitive to the protein antigen and a mean percent is determined. For use in the compositions of the invention, the candidate peptide or combination of candidate peptides has the following criteria: (1) the candidate peptide or combination of candidate peptides has a mean percent of at least about 10%, preferably at least about 20%, more preferably at least about 30%. more preferably at least about 40% and more preferably at least about 50-60% or greater; and (2) in the population of individuals tested at least about 60%, preferably at least about 75%, and more preferably at least about 90-100% have positive T call responses (S.I. equal to or greater than 2.0) in response to the candidate peptide or combination of candidate peptides. A candidate peptide or combination of candidate peptides meeting the above criteria is likely to comprise a sufficient percentage of the T cell reactivity to house dust mite protein allergen to induce T cell non-responsiveness or reduced T cell responsiveness in a substantial percentage of a population of individuals sensitive to house dust mite.

As an illustrative embodiment of the above-described algorithm, a set of overlapping peptides and candidate peptides derived from *Der p* I and *Der p* II respecifily were produced and tested. Secondary T cell cultures determined to be reactive with *Der p* I protein allergen were derived from 39 house dust mite-allergic subjects and analyzed for reactivity to an overlapping set of peptides, as well as candidate peptides derived from *Der p* I and *Der f* I protein allergen, DPI-21.2 (SEQ. ID. NO. 27), DFI-22.2 (SEQ. ID. NO. 28), DPI-23.31 (SEQ. ID. NO. 29) and DPI-26.6 (SEQ. ID. NO. 30), in an in vitro T cell proliferation assay as described herein. The results are shown in Fig. 3. The highest stimulation index greater than or equal to 2.0 in response to each peptide was recorded for each subject tested. The data were then analyzed by the equations above. The results and calculations of the percent of T cell reactivity for a single dust mite-allergic subject are shown below using formulas (1) and (2).

**T CELL REACTIVITY FOR PATIENT 1733**
**PEPTIDE STIMULATION INDEX**

| | |
|---|---|
| DPI-21.2 (SEQ. ID. NO. 27) | 3.6 |
| DPI-3 (SEQ. ID. NO. 3) | 3.9 |
| DPI-22.2 (SEQ. ID. NO. 28) | 3.1 |
| DPI-12.1 (SEQ. ID. NO. 6) | 2.2 |
| DPI-5.1 (SEQ. ID. NO. 7) | 3.5 |
| DPI-23.31 (SEQ. ID. NO. 29) | 5.7 |
| DPI-14 (SEQ. ID. NO. 9) | 2.3 |
| DPI-15 (SEQ. ID. NO. 10) | 2.8 |
| DPI-6.1 (SEQ. ID. NO. 11) | 2.1 |
| DPI-7.1 (SEQ. ID. NO. 12) | 2.2 |
| DPI-26.6 (SEQ. ID. NO. 30) | 5.0 |
| DPI-9 (SEQ. ID. NO. 14) | 2.4 |
| DPI-16 (SEQ. ID. NO. 15) | 2.0 |
| DPI-10 (SEQ. ID. NO. 16) | 0 |
| DPI-17 (SEQ. ID. NO. 17) | 0 |
| SUM OF STIMULATION INDICES | 40.8 (DENOMINATOR) |

% Reactivity of Peptide DPI-26.6 (SEQ. ID. NO. 30) for patient 1733 is

$$(1) \quad \frac{\text{DPI-26.6 (S.I.)}}{40.8} = \frac{5.0}{40.8} \quad X \quad 100 \quad = 12.3\%$$

$$(2) \quad \frac{DPI\text{-}7.1 + DPI\text{-}26.6 + DPI\text{-}9}{40.8} = \frac{2.2 + 5.0 + 2.4}{40.8} \times 100 = 24\%$$

Therefore the estimated range of T cell reactivity for Peptide DPI-26.6 (SEQ. ID. NO. 30) for this patient is 12.3%-24% of the total reactivity of the *Der p* I protein. The above calculation is repeated for any potential candidate peptides for each patient tested. In the population of 39 Cry j I-allergic subjects tested the following results were obtained:

| Candidate Peptides | Range of mean percentage T Cell Reactivity | Frequency of response at least one peptide |
| --- | --- | --- |
| DPI-21.2 (SEQ. ID. NO. 27), | | |
| DFI-22.2 (SEQ. ID. NO. 28) | | |
| DPI-23.31 (SEQ. ID. NO. 29), | | |
| DPI-26.6 (SEQ. ID. NO. 30) | 38-67% | 82% |

Thus, the combination of the four candidate peptides are well within the desired range for possessing, in combination, sufficient T cell reactivity of Group I protein allergen of *Der f* and *Der p,* and meet the first characteristic of a "unique" peptide of the invention.

The same calculation were determined for the Group II, *Der p* protein allergen. Secondary T cell cultures determined to be reactive with *Der p* I protein allergen were derived from 30 house dust mite-allergic subjects and analyzed for reactivity to an overlapping set of peptides, as well as candidate peptides derived from *Der p* II, DPII-20.9 (SEQ. ID. NO: 31), DPII-22.14 (SEQ. ID. NO. 32) and DPII-25.15 (SEQ. ID. NO. 33), in an in vitro T cell proliferation assay as described herein. The results are shown in Fig.4. The highest stimulation index greater than or equal to 2.0 in response to each peptide was recorded for each subject tested. The data were then analyzed by the equations above. In the population of 30 house dust mite-allergic subjects tested the following results were obtained:

| Candidate Peptides DPII-20.9 | Range of mean percentage T Cell Reactivity | Frequency of response at least one peptide |
| --- | --- | --- |
| (SEQ. ID. NO. 31), | | |
| DPII-22.14 (SEQ. ID. NO. 32) | | |
| DPII-25.15 (SEQ. ID. NO. 33) | 37-51% | 63% |

Thus, the combination of the three *Der p* II candidate peptides of the invention are well within the desired range for possessing, in combination, sufficient T cell reactivity of Group II house dust mite protein allergen of *Der p,* and meet the first characteristic of a "unique" peptide of the invention.

For the treatment of allergy in accordance with the methods of the invention, it is preferred that a peptide used in conjunction therewith does not bind immunoglobulin E (IgE) or binds IgE to a substantially lesser extent (i.e. at least 100-fold less binding and more preferably, at least 1,000-fold less binding) than the respective house dust mite protein allergen from which the peptide is derived binds IgE. The major complications of standard immunotherapy are IgE-mediated responses such as anaphylaxis. Immunoglobulin E is a mediator of anaphylactic reactions which result from the binding and cross-linking of antigen to IgE on mast cells or basophils and the release of mediators (e.g., histamine, serotonin, eosinophil chemotacic factors) in allergic ("atopic") patients. Thus, anaphylaxis in a substantial percentage of a population of individuals sensitive to the allergen being treated could be avoided by the use in immunotherapy of a peptide or peptides which do not bind IgE in a substantial percentage (e.g., at least about 75%) of a population of individuals sensitive to house dust mite allergen, or if the peptide binds IgE, such binding does not result in the release of mediators from mast cells or basophils. The risk of anaphylaxis could be reduced by the use in immunotherapy of a peptide or peptides which have reduced IgE binding. IgE binding may be tested, for example by direct ELISA or capture ELISA. Moreover, peptides which have minimal IgE stimulating activity are desirable for therapeutic effectiveness. Minimal IgE stimulating activity refers to IgE production that is less than the amount of IgE production and/or IL-4 production stimulated by the native respective house dust mite protein allergen. If a peptide binds IgE, it is preferable that such binding does not result in the release of mediators (e.g. histamines) from mast cells or basophils. To determine whether a peptide that binds IgE causes the release of mediators, a histamine release assay can be performed using standard reagents and protocols obtained for example, from Amac, Inc. (Westbrook, ME). Briefly, a buffered solution of a peptide to be tested is combined with an equal volume of whole heparinized blood from an allergic subject. After mixing and incubation, the cells are pelleted and the supernatants are processed and analyzed using a radio immunoassay to determine the amount of histamine released. Experiments to date indicate that candidate peptides DPI-21.2 (SEQ. ID. NO. 27), DFI-22.2 (SEQ. ID. NO. 28); DPI-23.31 (SEQ: ID. NO. 29). DPI-26.6 (SEQ. ID NO. 30), DPII-20.9 (SEQ. ID. NO. 31), DPII-22.14 (SEQ. ID. NO. 32) and DPII-25.15 (SEQ. ID. NO. 33) exhibit negative IgE reactivity or histamine release (data not shown).

The second characteristic for a unique peptide is that of "superior solubiiity" which was defined earlier as being solubility of greater than 3 mg/ml at a pH in a range of pH 6 to pH 8. Solubility in a physiologically acceptable pH range (e.g. pH 6 to pH 8) is particularly important when formulating a multipeptide therapeutic for injection. Administration of a soluble drug product in a physiologically acceptable pH range by intravenous or subcutaneous injection provides about 100% bioavailability of the drug component to the physiological system into which the drug is being introduced. Thus, it is necessary that a drug product intended for injection be fluid to the extent that easy syringability exists, and the active component be soluble as well if maximum therapeutic effect is to be achieved. Solubility is also useful when formulating compositions to be administered via other modes of administration such as by oral administration (tablet, aerosol, sublingual), or sustained release preparations and formulations.

Proteins and peptides may be difficult to formulate into soluble compositions as a peptide may not be soluble in any desirable pH range or may be soluble in only a narrow pH range. It is particularly difficult when multiple peptides are being formulated together into a single multipeptide formulation, as each peptide may be soluble in a pH range which does not overlap with those of the other peptides in the formulation. As a result, it is the requirement of "superior solubility" which requires the most formulation flexibility in that considerable modification of the targeted candidate peptides may be necessary to successfully formulate a multipeptide drug product.

Some of the unique peptides of the invention are the product of multiple amino acid modifications of the original targeted candidate peptide sequence ("parent") from which the modified unique peptides of the invention were originally derived. Such modified "unique" peptides of use in the invention include DPI-23.31 (SEQ ID NO:29), DPI-26.6 (SEQ. ID. NO. 30). DPII-20.9 (SEQ. ID. NO: 31), DPII-22.14 (SEQ. ID. NO. 32) and DPII-25.15 (SEQ. ID. NO. 33) all as shown in Fig. 1. For example, the amino acid sequence of DpII-22.14 (SEQ. ID. NO. 32) was derived from the protein sequence of *Der p* II by first identifying those regions of the native protein with high T-cell reactivity using a set of overlapping peptides that covered the entire protein sequence. Several peptides were found to exhibit high T-cell reactivity, corresponding to three different locations/areas in the protein. One of these areas were covered by three adjacent peptides each being about 25-29 amino acids long and with 10-15 amino acids overlap, DpII-3.1 (SEQ. ID. NO. 20), DpII-4 (SEQ. ID. NO. 21)and DpII-5 (SEQ. ID. NO. 22) (Fig. 2) that all exhibited high T-cell reactivity. Based on this T-cell map a new peptide was synthesized, with the amino acid sequence QLEAVFEANQNTKTAKIEIKASIDGLEV (SEQ. ID. NO. 34), 28 amino acids long, which exhibits most of the reactivity found in the individual peptides (data not shown) and contains all of DpII-4 (SEQ. ID. NO. 21)and parts of DpII-3.1 (SEQ. ID. NO. 20) and DpII-5 (SEQ. ID. NO. 22). However, this peptide did not meet the "superior solubility" requirement of 3 mg/ml for a "unique peptide of the invention nor did it meet the stability requirement. Therefore two analogs were made with addition of one or two charged residues at each end, DpII-22.2 **K**QLEAVFEANQNTKTAKIEIKASIDGLEV**K** (SEQ. ID. NO. 35) and DpII-22.6 **DK**QLEAVPEANQNTKTAKIEIKASIDGLEVD (SEQ. ID. NO. 36) and the stability improved substantially but the solubility did not reach the standard of a "superior solubility". In the second attempt a series of truncated analogs with up to five charged amino acid residues added to the ends (Table I).

Table 1

| DpII-22 (SEQ. ID. NO. 34) | QLEAVFEANQNTKTAKIEIKASIDGLEV |
|---|---|
| DpII-22.2 (SEQ. ID. NO. 35) | **K**QLEAVFEANQNTKTAKIEIKASIDGLEV**K** |
| DpII-22.6 (SEQ. ID. NO. 36) | **DK**QLEAVPEANQNTKTAKIEIKASIDGLEVD |
| DpII-22.4 (SEQ. ID. NO. 37) | QLEAVFEANQNTKTAKIEIKASID**E** |
| DpII-22.5 (SEQ. ID. NO. 38) | **DK**QLEAVFEANQNTKTAKIEIKASID**E** |
| DpII-22.8 (SEQ. ID. NO. 39) | **DKE**QLEAVFEANQNTKTAKIEIKASID**E** |

(continued)

| | |
|---|---|
| DpII-22.9 (SEQ. ID. NO. 40) | **DKE**QLEAVFEANQNTKTAKIEIKASID**EE** |
| DpII-22.12 (SEQ. ID. NO. 41) | **DKE**QLEAVFEANQATKTAKIEIKASID**E** |
| DpII-22.16 (SEQ. ID. NO. 42) | **DKEQ**LEAVFEANQNTKTAKIRIKAS**E** |
| DpII-22.19 (SEQ. ID. NO. 43) | **DKE**QLEAVFEANQNTKTAKIEIKA**D** |
| DpII-22.20 (SEQ. ID. NO. 44) | **DKE**LEAVFEANQNTKTAKIEIKAK |
| DpII-22.21 (SEQ. ID. NO. 45) | **DKE**LEAVFANQNTKTAKIEI**ED** |
| DpII-22.22 (SEQ. ID. NO. 46) | **DKE**LEAVFEANQNTKTAKIEKK |
| DpII-22.26 (SEQ. ID. NO. 47) | **DKE**LEAVFEANQNTKTAKIEIK |
| DpII-22.23 (SEQ. ID. NO. 48) | **DKE**LEAVFEANQNTKTAKI**ED** |
| DpII-22.24 (SEQ. ID. NO. 49) | **DKE**LEAVFEANQNTKTAKIE**K** |
| DpII-22.25 (SEQ. ID. NO. 50) | **DKE**LEAVFEANQNTKTAKIE |
| DpII-22.14 (SEQ. ID. NO. 32) | **DKE**LEAVFEANQNTKTAK**A**E |
| DpII-22.10 (SEQ. ID. NO. 51) | LEAVFEANQNTKTAK |
| DpII-22.11 (SEQ. ID. NO. 52) | LEAVFEANQ**A**TKTAK |
| DpII-22.18 (SEQ. ID. NO. 53) | DKTAKIEIKASIDGLE |
| DpII-22.15 (SEQ. ID. NO. 54) | KTAKIEIKASIDGLE |

Of these the DpII-22.5, DKQLEAVFEANQNTKTAKIEIKASIDE (SEQ. ID. NO. 38), was promising since it retained the T-cell reactivity of the "parent" peptide, DPII-22, (SEQ. ID. NO. 34)and was very soluble, but it was very difficult to synthesize. The difficulties in synthesizing this sequence were found to disappear with the replacement of the hydrophobic isoleucine with the less hydrophobic alanine and simultaneously the solubility increased by an order of magnitude. Peptide DpII-22.14, DKELEAVFEANQNTKTAKAE (SEQ. ID. NO. 32), was found to possess almost the same T-cell reactivity as the "parent" peptide DpII-22 (SEQ. ID. NO. 34)as well as being soluble at greater than 3 mg/ml at a pH in the pH range 6.0 to 8.0, and was easy to synthesize and purify. Therefore, DPB-22 (SEQ. ID. NO. 34) was chosen as a "unique" peptide when it was determined to be stable at a pH in the range of 6.0 to 8.0. The development of the other modified "unique" peptides, DpI-23.31 (SEQ. ID. NO. 29), DpI-26.6 (SEQ. ID. NO. 30) and DpII-25.15 (SEQ. ID. NO. 33), followed a process similar to that described above for DpII-22.14 (SEQ. ID. NO. 32). Peptides DPII-22.14 (SEQ. ID. NO. 32), DpI-23.31 (SEQ. ID. NO. 29), DpI-26.6 (SEQ. ID. NO. 30) and DpII-25.15 (SEQ. ID. NO. 33), all as shown in Fig. 1 are novel modified peptides of this invention.

The third criteria which the unique peptides of this invention must meet is stability, particularly solution stability, in a physiologically acceptable pH range of pH 6 to pH 8. It must be stable under the conditions of manufacture and storage, and under conditions of reconstitution if necessary. Stability testing establishes the time period for which the integrity, quality and purity of the drug product is preserved in its finished dosage form. Stability testing may be performed concurrently with solubility studies as discussed in Example 3. An equal concentration composition comprising each of the candidate peptides of the invention remained stable (e.g. no significant degradation) in solution at a common "window" within the pH range from pH 6-pH 8 at about room temperature and at about 5°C for at least 24 hours (see, Fig. 6).

Therefore, candidate peptides DPI-21.2 (SEQ. ID. NO. 27), DFI-22.2 (SEQ. ID. NO. 28), DPI-23.31 (SEQ. ID. NO. 29), DPI-26.6 (SEQ. ID. NO. 30), DPII-20.9 (SEQ. ID. NO. 31), DPII-22.14 (SEQ. ID. NO. 32) and DPII-25.15 (SEQ. ID. NO. 33) possess each of the three required "unique" characteristics outlined above, indicating that this combination of peptides is suitable for formulation as an optimized therapeutic drug product for administration to humans for treatment of allergy to house dust mite allergen.

Highly purified peptides of this invention, may be produced synthetically by chemical synthesis using standard techniques. Various methods of chemically synthesizing peptides are known in the art such as solid phase synthesis which has been fully or semi automated on commercially available peptide synthesizers. Synthetically produced peptides may then be purified to homogeneity (i.e. at least 90%, more preferably at least 95% and even more preferably at least 97% purity), free from all other polypeptides and contaminants using any number of techniques known in the literature for protein purification.

In accordance with one procedure for producing highly purified homogenous peptides of the invention, a peptide produced by synthetic chemical means (either anchored to a polymer support "solid phase synthesis" or by conventional homogenous chemical reactions "solution synthesis") may be purified by preparative reverse phase chromatography. In this method, the synthetically produced peptide in "crude" form is dissolved in an appropriate solvent (typically an aqueous buffer) and applied to a separation column (typically a reverse phase silica based media, in addition, polymer or carbon based media may be used). Peptide is eluted from the column by increasing the concentration of an organic component (typically acetonitrile or methanol) in an aqueous buffer (typically TFA, triethylamine phosphate, acetate or similar buffer). Fractions of the eluate will be collected and analyzed by appropriate analytical methods (typically reverse phase HPLC or CZE chromatography). Those fractions having the required homogeneity will be pooled. The counter ion present may be changed by additional reverse phase chromatography in the salt of choice or by ion exchange resins. The peptide may then be isolated as its acetate or other appropriate salt. The peptide is then filtered and the water removed (typically by lyophilization) to give a homogenous peptide composition containing at least 90%, more preferably at least 95% and even more preferably at least 97% of the required peptide component. Optionally, or in conjunction with reverse phase HPLC as described above, purification may be accomplished by affinity chromatography, ion exchange, size exclusion, counter current or normal phase separation systems, or any combination of these methods. Peptide may additionally be concentrated using ultra filtration, rotary evaporation, precipitation, dialysis or other similar techniques.

The highly purified homogenous peptide composition is then characterized by any of the following techniques or combinations thereof: a) mass spectroscopy to determine molecular weight to check peptide identity; b) amino acid analysis to check the identity of the peptide via amino acid composition; c) amino acid sequencing (using an automated protein sequencer or manually) to confirm the defined sequence of amino acid residues; d) HPLC (multiple systems if desired) used to check peptide identity and purity (i.e. identifies peptide impurities); e) water content to determine the water concentration of the peptide compositions; f) ion content to determine the presence of salts in the peptide composition; and g) residual organics to check for the presence of residual organic reagents, starting materials, and/or organic contaminants.

A peptide of the invention may also be produced by recombinant DNA techniques in a host cell transformed with a nucleic acid sequence coding for such peptide. When produced by recombinant techniques, host cells transformed with nucleic acid encoding the desired peptide are cultured in a medium suitable for the cells and isolated peptides can be purified from cell culture medium, host cells, or both using techniques known in the art for purifying peptides and proteins including ionexchange chromatography, ultra filtration, electrophoresis or immunopurification with antibodies specific for the desired peptide. Peptides produced recombinantly may be isolated and purified to homogeneity, free of cellular material, other polypeptides or culture medium for use in accordance with the methods described above for synthetically produced peptides.

In certain limited circumstances, peptides of this invention may also be produced by chemical or enzymatic cleavage of a highly purified full length or native protein of which the sites of chemical digest or enzymatic cleavage have been predetermined and the resulting digest is reproducible. Peptides having defined amino acid sequences can be highly purified and isolated free of any other poly peptides or contaminants present in the enzymatic or chemical digest by any of the procedures described above for highly purified, and isolated synthetically or recombinantly produced peptides.

The present invention also pertains to therapeutic compositions and multipeptide therapeutic formulations comprising the unique peptides of the invention. Therapeutic compositions of the invention may comprise one or more of the unique peptides of the invention which may be administered simultaneously or sequentially as single treatment episode for treatment of allergy to house dust mite allergen in a human or other mammal. Such a treatment regimen may not necessarily be a physical mixture of more than one peptide of the invention, but does comprise a combination of such peptides administered simultaneously or sequentially as a single treatment episode in order to achieve the maximum therapeutic effect the combination of the unique peptides, DPI-21.2 (SEQ. ID. NO. 27), DFI-22.2 (SEQ. ID. NO. 28), DPI-23.31 (SEQ. ID. NO. 29), DPI-26.6 (SEQ. ID. NO. 30), DPII-20.9 (SEQ. ID. NO. 31), DPII-22.14 (SEQ. ID. NO. 32) and DPII-25.15 (SEQ. ID. NO. 33) provide (e.g. solubility and stability in acceptable physiological pH range as well as having a range of T cell reactivity of 38-67% of the total T cell reactivity of the Group I house dust mite protein allergen and a frequency of response of 91 % for at least one *Der p* I peptide tested in a population of individuals allergic to house dust mite and similarly, have a range of T cell reactivity of 37-51% of the total T cell reactivity of the Group II house dust mite protein allergen and a frequency of response of 63% for at least one *Der p* II peptide tested in a population of individuals allergic to house dust mite).

Therapeutic compositions of the invention comprise one or more of peptides DPI-21.2 (SEQ. ID. NO. 27), DFI-22.2 (SEQ. ID. NO. 28), DPI-23.31 (SEQ. ID. NO. 29), DPI-26.6 (SEQ. ID. NO. 30), DPII-20.9 (SEQ. ID. NO. 31), DPII-22.14 (SEQ. ID. NO. 32) and DPII-25.15 (SEQ. ID. NO. 33) also comprise one or more pharmaceutically acceptable carriers such as excipients which are compatible with peptide or peptides present in a single composition. When the composition is a multipeptide formulation, the pharmaceutically acceptable carrier must be compatible with all of the peptides in the multipeptide formulation. Preferred excipients include but are not limited to sterile water, sodium phosphate, mannitol, or both sodium phosphate and mannitol or any combination thereof. Other suitable excipients include but are not limited to sorbitol, sucrose, dextrose, lactose dextran and PVP. Additionally, due to the potential for dimerization of the peptides in a mutlipeptide formulation, there may also be included an agent such as EDTA to prevent dimerization. Alternatively, any other material or procedures known in the art to prevent dimerization may be used. In addition, pharmaceutically acceptable counter ions may be added during the preparation of the multipeptide formulation. Examples of pharmaceutically acceptable counter ions include acetate, HCl, and citrate.

A therapeutic composition of the invention should be sterile, stable under conditions of manufacture, storage, distribution and use and should be preserved against the contaminating action of microorganisms such as bacteria and fungi. A preferred means for manufacturing a therapeutic compositions of the invention in order to maintain the integrity of the composition (i.e. prevent contamination, prolong storage, etc.) is to prepare the formulation of peptide and pharmaceutically acceptable carrier(s) such that the composition may be in the form of a lyophilized powder which is reconstituted in a pharmaceutically acceptable carrier, such as sterile water, just prior to use. In the case of sterile powders for the preparation of sterile injectable solutions, the preferred methods of preparation are vacuum drying, freeze-drying or spin drying which yields a powder of the active ingredient plus any additional desired ingredient from a previously sterile-filtered solution thereof.

A preferred multipeptide formulation comprises the following unique peptides DPI-21.2 (SEQ. ID. NO. 27), DFI-22.2 (SEQ. ID. NO. 28), DPI-23.31 (SEQ. ID. NO. 29), DPI-26.6 (SEQ. ID. NO. 30), DPII-22.14 (SEQ. ID. NO. 32) and DPII-25.15 (SEQ. ID. NO. 33) and sodium phosphate and mannitol, and optionally may further comprise DPII-20.9 (SEQ. ID. NO. 31). For this embodiment, EDTA is added to the formulation. A suitable counter ion such as acetate may also be added during the preparation of the formulation. The formulation is preferably prepared in the form of a lyophilized powder which is reconstituted in a physiologically acceptable carrier, such as sterile water, prior to use. Several non-limiting examples of a preferred multipeptide formulations of this invention are described below. The unique house dust mite protein allergen peptides will preferably be combined during manufacturing with the appropriate counter ion to produce one vial containing a sterile, pyrogen free, and preferably lyophilized powder of the desired formulation:

**Active**: *Der p* I, *Der f*l and *Der p* II peptides DPI-21.2 (SEQ. ID. NO. 27), DFI-22.2 (SEQ. ID. NO. 28), DPI-23.31 (SEQ. ID. NO. 29), DPI-26.6 (SEQ. ID. NO. 30), DPII-22.14 (SEQ. ID. NO. 32) and DPII-25.15 (SEQ. ID. NO. 33) In concentration of 0.75 mg per peptide

**Inactives:** 0.05 M Sodium Phosphate U.S.P.
5% w/v Mannitol, U.S.P.
0.1 mg/ml EDTA disodium dihydrate U.S.P.
Final pH 7.2 -7.4

**Diluent**: Sterile Water for Injection, U.S.P.

**Optionally** 0.75 mg of DPII-20.9 (SEQ. ID. NO. 31) may be added to the active ingredients.

A preferred combination of multipeptide formulations suitable for administration simultaneously or sequentially as a single treatment episode and contained in two separate sterile, pyrogen free vials preferably in the form of lyophilized powders include the following formulations:

**Vial #1**

**Active**: *Der p* I, *Der f*l and *Der p* **II** peptides DFI-22.2(SEQ. ID. NO. 28), DPI-23.31 (SEQ. ID. NO. 29), and DPH-22.14 (SEQ. ID. NO. 32)
In concentration of 0.75 mg per peptide

**Inactives:** 0.05 M Sodium Phosphate U.S.P.
5% w/v Mannitol, U.S.P.
0.1 mg/ml EDTA disodium dihydrate
Final pH 7.0

**Diluent**: Sterile Water for Injection, U.S.P.

Vial #2

**Active:** *Der p* I, and *Der p* II peptides DPI-21.2 (SEQ. ID. NO. 27), DPI-26.6 (SEQ. ID. NO. 30), DPII 20.9 (SEQ. ID. NO. 31), DPII-25.15 (SEQ. ID. NO. 33)

In concentration of 0.75 mg per peptide

**Inactives**: 0.05 M Sodium Phosphate U.S.P.

5% w/v Mannitol, U.S.P.

0.1 mg/ml EDTA disodium dihydrate U.S.P.

Final pH 6.2

**Diluent**: Sterile Water for Injection, U.S.P.

The multipeptide formulations of the invention may also be provided in the form of a kit, including instructions for use.

Administration of the therapeutic compositions and multipeptide formulations described above to an individual, preferably in non-immunogenic form, can be carried out using known procedures at dosages and for periods of time effective to cause down regulation of the house dust mite-specific immune response (i.e., reduce the allergic symptoms caused by house dust mite protein allergen of the individual. Down regulation of the allergic immune response to house dust mite allergen in humans may be determined clinically whenever possible (see e.g., Varney et al, *British Medical Journal*, **302**:265-269 (1990), or may be determined subjectively (i.e. the patient feels as if some or all of the allergic symptoms caused by house dust mite allergens have been alleviated).

One of the unique characteristics of each unique peptide in accordance with the invention is that each peptide possesses "superior solubility". Therefore, compositions and multipeptide formulations of the invention are particularly suitable for administration by injection (e.g. subcutaneous, or intravenous). However, optimized compositions and multipeptide formulations of the invention may be administered in any convenient manner wherein solubility of the active drug component is either desirable or acceptable, such as by injection (subcutaneous, intravenous, etc.), oral administration, sublingual, inhalation, transdermal application, rectal administration, or any other route of administration known in the art for administering soluble therapeutic agents. It may be desirable to administer simultaneously or sequentially a therapeutically effective amount of one or more of the therapeutic compositions of the invention to an individual as a single treatment episode. Each of such compositions for administration simultaneously or sequentially as a single treatment episode, may comprise only one unique peptide of the invention or may comprise an optimized multipeptide formulation in accordance with the invention.

For subcutaneous injection of one or more therapeutic compositions and multipeptide formulations of the invention, preferably about 1 $\mu$g- 3 mg and more preferably from about 20$\mu$g-1.5 mg, and even more preferably about 50 $\mu$g- 750 $\mu$g of each active component (peptide) per dosage unit may be administered. It is especially advantageous to formulate parenteral compositions in unit dosage form for ease of administration and uniformity of dosage. Unit dosage form as used herein refers to physically discrete units suited as unitary dosages for human subjects to be treated; each unit containing a predetermined quantity of active compound calculated to produce the desired therapeutic effect in association with the desired pharmaceutical carrier. The specification for the novel unit dosage forms of the invention are dictated by and directly dependent on (a) the unique characteristics of the active compound and the particular therapeutic effect to be achieved, and (b) the limitations inherent in the art of compounding such an active compound for the treatment of human subjects.

To administer a composition of the invention by other than parenteral administration, (i.e. by oral administration) it may be necessary to coat the composition with, or co-administer the composition with, a material to prevent its inactivation or enhance its absorption and bioavailability. For example, a peptide formulation may be co-administered with enzyme inhibitors or in liposomes. Enzyme inhibitors include pancreatic trypsin inhibitor, diisopropylfluorophosphate (DEP) and trasylol. Liposomes include water-in-oil-in-water CGF emulsions as well as conventional liposomes (Strejan et al., (1984) J. Neuroimmunol., 7:27). When a peptide is suitably protected, the peptide may be orally administered, for example, with an inert diluent or an assimilable edible carrier. The peptide and other ingredients may also be enclosed in a hard or soft shell gelatin capsule, compressed into tablets, or incorporated directly into the individual's diet. For oral therapeutic administration, the active compound may be incorporated with excipients and used in the form of ingestible tablets, buccal tablets, troches, capsules, elixirs, solutions, gels, suspensions, syrups, wafers, and the like. Such compositions and preparations should contain at least 1% by weight of active compound. The percentage of the composition and preparations may, of course, be varied and may conveniently be between about 5 to 80% of the weight of the unit. The amount of active compound in such therapeutically useful compositions is such that a suitable dosage will be obtained. In addition, the active compound may be incorporated into sustained-release or controlled release (steady state or pulsatile release) preparations and formulations.

Effective amounts of the optimized drug compositions of the invention will vary according to factors such as the degree of sensitivity of the individual to the antigen, the age, sex, and weight of the individual, and the ability of peptide to cause down regulation of the antigen specific immune response in the individual. Dosage regimen may be adjusted to provide the optimum therapeutic response. For example, several divided doses may be administered over the course of days, weeks, months or years, or the dose may be proportionally increased or reduced with each subsequent injection as indicated by the exigencies of the therapeutic situation. In one preferred therapeutic regimen, subcutaneous injections of therapeutic compositions are given once a week for 3-6 weeks. The dosage may remain constant for each injection or may increase or decrease with each subsequent injection. A booster injection may be administered at intervals of about three months to about one year after initial treatment and may involve only a single injection or may involve another series of injections similar to that of the initial treatment.

This invention is illustrated by the following non-limiting examples.

**Example 1**

**Determination of the percentage of total house dust mite Group I T cell reactivity and patient coverage of the combination of unique *Der p* I and *Der f* I peptides.**

Synthesis of Overlapping Peptides

*Der p* I was divided into a set of 17 overlapping peptides. Overlapping peptides and *Der p* I and *Der f* I unique peptides (i.e. DPI-21.2 (SEQ. ID. NO. 27), DFI-22.2 (SEQ. ID. NO. 28), DPI-23.31 (SEQ. ID. NO. 29), DPI-26.6 (SEQ. ID. NO. 30)) were synthesized using standard Fmoc/tBoc synthetic chemistry and purified by Reverse Phase HPLC. Figure 1 shows the unique *Der p* I, and *Der f* I peptides and Figure 2 shows the overlapping *Der p* I peptides used in these studies. The peptide names are consistent throughout.

T cell responses to Group I overlapping peptides and unique peptides

Peripheral blood mononuclear cells (PBMC) were purified by Ficoll-Hypaque centrifugation of 60 ml of heparinized peripheral blood from house dust mite-allergic individuals who exhibited clinical symptoms of mite allergy and who were skin test positive for house dust mite.

$10^7$ PBMC from each patient were cultured in 5 ml RPMI-1640 containing 5% pooled human AB serum and supplemented with glutamine, penicillin, streptomycin and HEPES buffer in the presence of 20 $\mu$g/ml purified native *Der p* I/ml at 37°C for 6 days. Viable cells were then purified by Ficoll-Hypaque centrifugation and cultured for 2-3 additional weeks in RPMI-1640/5% AB serum containing 5 units recombinant human IL-2/ml and 5 units recombinant human EL-4/ml. The resting T cells were then tested in a secondary proliferation assay to assess T cell responses to purified native *Der p* I, overlapping peptides and unique peptides. For assay, 2 X $10^4$ resting T cells were cultured in 200 $\mu$l of RPMI-1640/5% AB serum for 3 days at 37°C in the presence of 2 X $10^4$ autologous Epstein-Barr virus transformed B cells (20,000 Rads) or in the presence of 5 X $10^4$ PBMC (3500 Rads) as antigen presenting cells with various concentrations of purified native *Der p* I or synthetic *Der p* I unique peptide or overlapping peptides. Each well then received 1 $\mu$Ci tritiated thymidine for 16 hours. The counts incorporated were collected onto glass fiber filters and processed for liquid scintillation counting. Medium alone, acting as negative control, contained no allergen or peptide. The results are shown in Fig. 3. The highest stimulation index greater than or equal to 2.0 in response to each peptide was recorded for each subject tested. The data were then analyzed by the equations described earlier in the specification.

The combination of Group I candidate peptides DPI-21.2 (SEQ. ID. NO. 27), DFI-22.2 (SEQ. ID. NO. 28), DPI-23.31 (SEQ. ID. NO. 29) DPI-26.6 (SEQ. ID. NO. 30), had a range of T cell reactivity of about 38-67% based on an analysis of 39 patients, the frequency of response at 82% represents reactivity to at least one of the candidate peptides, indicating that this combination of peptides fits the first criteria for "unique" peptides of the invention in that the combination of peptides DPI-21.2 (SEQ. ID. NO. 27), DFI-22.2 (SEQ. ID. NO. 28), DPI-23.31 (SEQ. ID. NO. 29), DPI-26.6 (SEQ. ID. NO. 30), comprise a sufficient percentage of the total T cell reactivity to Group I protein allergens from *Der p* and *Der f* in a substantial percentage of the population tested.

**Example 2**

**Determination of the percentage of total house dust mite Group II T cell reactivity and patient coverage of the combination of unique *Der p* I candidate peptides**

Synthesis of Overlapping Peptides

*Der p* II was divided into a set of 9 overlapping peptides. Overlapping Der *p* II peptides and *Der p* II unique peptides (i.e. DPII-20.9 (SEQ. ID. NO. 31), DPII-22.14 (SEQ. ID. NO. 32), and DPII-25.15 (SEQ. ID. NO. 33)) were synthesized using standard Fmoc/tBoc synthetic chemistry and purified by Reverse Phase HPLC. Figure 1 shows the unique *Der p* II peptides and Figure 2 shows the overlapping *Der p* II peptides used in these studies. The peptide names are consistent throughout.

T cell responses to Group II overlapping peptides and unique candidate peptides

Secondary *Der p* II reactive T cell cultures derived from 30 mite-allergic patients were analyzed for reactivity to an overlapping set of *Der p* II peptides and *Der p* II candidate peptides. in an in vitro T cell proliferation assay as described in Example 1. The results are shown in Fig. 4. The highest stimulation index greater than or equal to 2.0 in response to each peptide was recorded for each subject tested. The data were then analyzed by the equations described earlier in the specification.

The combination of candidate peptides DPII-20.9 (SEQ. ID. NO. 31), DPII-22.14 (SEQ. ID. NO. 32), and DPII-25.15 (SEQ. ID. NO. 33), had a range of T cell reactivity of about 36%-51 % based on an analysis of 30 patients (Fig. 4). The frequency of response to at least one of the candidate peptides was about 63%, indicating that this combination of peptides fits the first criteria for "unique" peptides of the invention, in that the combination of *Der p* II peptides DPII-20.9 (SEQ. ID. NO. 31), DPII-22.14 (SEQ. ID. NO. 32), and DPII-25.15 (SEQ. ID. NO. 33), comprise a sufficient percentage of the total T cell reactivity to *Der p* II in a substantial percentage of the population tested.

**Example 3**

**Determination of pH-solubility and pH-stability profiles of candidate peptides of the invention**

1. Buffer Preparation

50 mM sodium phosphate stock solutions:

Stock solution A: 0.66 g (0.05 mol) of monobasic sodium phosphate monohydrate U.S.P. and 50 mg EDTA disodium dihydrate, U.S.P. were dissolved in 100 mL of WFT. The solution was filtered through a 0.2 micron filter

Stock solution B: 0.71 g (0.05 mol) of dibasic sodium phosphate U.S.P. were disolved in 100ml WFI. The solution was filtered through a 0.2 micron filter.

2. Initial peptide dispersions

Dispersion A: 3.0 mg of each candidate peptide,DPI-21.2 (SEQ. ID. NO. 27), DFI-22.2 (SEQ. ID. NO. 28), DPI-23.31 (SEQ. ID. NO. 29), DPI-26.6 (SEQ. ID. NO. 30), DPII-20.9 (SEQ. ID. NO. 31), DPII-22.14 (SEQ. ID. NO. 32) and DPII-25.15 (SEQ. ID. NO. 33), was weighed out separately and placed in separate 1.5 mL eppendoff vials with 600 $\mu$L of stock solution A. The composition was agitated for 5 seconds to mix well.

Dispersion B: 3.0 mg of each peptide, DPI-21.2 (SEQ. ID. NO. 27), DFI-22.2 (SEQ. ID. NO. 28), DPI-23.31 (SEQ. ID. NO. 29), DPI-26.6 (SEQ. ID. NO. 30), DPII-20.9 (SEQ. ID. NO. 31), DPII-22.14 (SEQ. ID. NO. 32) and DPII-25.15 (SEQ. ID. NO. 33), was weighed out separately and placed in separate 1.5 mL eppendoff vials with 600 $\mu$L of stock solution B. The mixture was agitated for 5 seconds to mix well.

Dispersions A and B were sonicated for 2 minutes for good homogeneity. A small volume was pipetted from each dispersion into a labeled eppendoff vial according to the following volume ratio:

| Vial # | Suspension A ($\mu$L) | Suspension B ($\mu$L) | Total Volume ($\mu$L) | Estimated final pH |
|--------|--------|--------|--------|--------|
| 1 | 100 | 0 | 100 | 5.2 |
| 2 | 80 | 20 | 100 | 6.2 |
| 3 | 60 | 40 | 100 | 6.6 |
| 4 | 40 | 60 | 100 | 6.8 |
| 5 | 20 | 80 | 100 | 7.1 |
| 6 | 0 | 100 | 100 | 8.0 |

The resultant solutions/suspensions were stored in the dark at about 22°C $\pm$ 2 for 24 hours without agitation. The solutions were filtered and filtrates were analyzed for pH and peptide concentration.

The concentration of filtered peptide solutions was determined by HPLC analysis. In this experiment, the solubility of peptide at each pH is defined as the amount of peptide remaining in solution after filtration through a membrane filter having a 0.2 micrometer pore size. The extent of degradation of peptides was estimated by calculating the percent of total degradant peak area over the total peak area.

The pH values with respect to the solubility values was plotted and are shown in Fig. 5 for each respective peptide. As shown in the solubility curve for each peptide as represented in Fig. 5, peptides DPI-21.2 (SEQ. ID. NO. 27), DFI-22.2 (SEQ. ID. NO. 28), DPI-23.31 (SEQ. ID. NO. 29), DPI-26.6 (SEQ. ID. NO. 30), DPII-20.9 (SEQ. ID. NO. 31), DPII-22.14 (SEQ. ID. NO. 32) and DPII-25.15 (SEQ. ID. NO. 33) are each soluble at greater than 3 mg/ml at a pH in the pH range of pH 6 to pH 8.

The pH-stability profiles for each peptide DPI-21.2 (SEQ. ID. NO. 27), DFI-22.2 (SEQ. ID. NO. 28), DPI-23.31 (SEQ. ID. NO. 29), DPI-26.6 (SEQ. ID. NO. 30), DPII-20.9 (SEQ. ID. NO. 31), DPH-22.14 (SEQ. ID. NO. 32) and DPII-25.15 (SEQ. ID. NO. 33) in equal concentration combination at 3, 2, and 1 mg/ml per peptide and prepared in duplicate as described above with one set stored at about 5° C for 24 hours and one set stored at about 22°C ± 2 for 24 hours, were calculated and tabulated as a function of the percentage of total of degradant peak area (Fig. 6). As can be seen from the data in Fig. 6, the percent degradation of peptide at each concentration was less for peptides stored at about 5°C for 24 hours as compared to peptides stored at 22° C ±2 for 24 hours over the critical pH range of pH 6.0 to pH 8.0 However, acceptable solution stability is demonstrated for all the peptides in a common "window" within the pH range of pH 6 to pH 8 at either temperature.

Therefore, each of peptides DPI-21.2 (SEQ. ID. NO. 27), DFI-22.2 (SEQ. ID. NO. 28), DPI-23.31 (SEQ. ID. NO. 29), DPI-26.6 (SEQ. ID. NO. 30), DPII-20.9 (SEQ. ID. NO. 31), DPII-22.14 (SEQ. ID. NO. 32) and DPII-25.15 (SEQ. ID NO. 33) was determined to possess the appropriate solubility and stability required of a unique peptide for use in a composition of the invention.

**Claims**

1. A therapeutic composition comprising at least one isolated peptide selected from the group consisting of: DPI-21.2 (SEQ ID NO:27), and DFI-22.2 (SEQ ID NO:28) and at least one peptide selected from the group consisting of DPI-23.31 (SEQ ID NO:29), DPI-26.6 (SEQ ID NO: 30), DPII-20.9 (SEQ ID NO:31), DPII-22.14 (SEQ ID NO:32) and DPII-25.15 (SEQ ID NO:33).

2. A therapeutic composition comprising:

a) peptides DFI-22.2 (SEQ ID NO:28); DPI-23.1 (SEQ ID NO:29) and DPII-22.14 (SEQ ID NO:32);
b) peptides DPI-21.2 (SEQ ID NO:27), DPI-26.6 (SEQ ID NO:30), and DPII-25.15 (SEQ ID NO:33);
c) peptides DPI-21.2 (SEQ ID NO:27), DFI-22.2 (SEQ ID NO:28), DPI-23.31 (SEQ ID NO:29), DPI-26.6 (SEQ

ID NO:30), DPII-22.14 (SEQ ID NO:32) and DPII-25.15 (SEQ ID NO:33); or

d) peptides DPI-21.2 (SEQ ID NO:27), DFI-22.2 (SEQ ID NO:28), DPI-23.31 (SEQ ID NO:29), DPI-26.6 (SEQ ID NO:30), DPII-20.9 (SEQ ID NO:31), DPII-22.14 (SEQ ID NO:32) and DPII-25.15 (SEQ ID N0:33).

**3.** A composition as claimed in claim 1 or claim 2 for use in therapy.

**4.** A composition as claimed in claim 1 or claim 2 for use in:

a) a method of treating sensitivity to house dust mite allergen in a mammal comprising administering simultaneously, or sequentially at least two different compositions of claim 1; or

b) a method of treating sensitivity to house dust mite allergen in a mammal comprising administering a therapeutic composition of claim 2.

**5.** A multipeptide formulation for pharmaceutical administration comprising:

a) at least one isolated peptide selected from the group consisting of DPI-21.2 (SEQ ID NO:27), and DFI-22.2 (SEQ ID NO:28) and at least one peptide selected from the group consisting of DPI-23.31 (SEQ ID NO:29), DPI-26.6 (SEQ ID NO:30), DPII-20.9 (SEQ ID NO:31), DPII-22.14 (SEQ ID NO:32) and DPII-25.15 (SEQ ID NO:33), each peptide being soluble and stable at a pH in the range of pH 6.0 to pH 8.0 and wherein said formulation comprises a sufficient percentage of T cell activity of Group I and Group II *Der f* and *Der p* house dust mite allergens;

b) a pharmaceutically acceptable carrier; and optionally

c) one or more of EDTA and a pharmaceutically acceptable counter ion.

**6.** A formulation as claimed in claim 5, which comprises at least 37% of T cell activity of Group I and Group II *Der f* and *Der p* house dust mite allergens.

**7.** The multipeptide formulation of claim 5 or claim 6 comprising:

a) peptides DPI-21.2 (SEQ ID NO:27), DFI-22.2 (SEQ ID NO:28), DPI-23.31 (SEQ ID NO:29), DPI-26.6 (SEQ ID NO:30), DPII-22.14 (SEQ ID NO:32) and DPII-25.15 (SEQ ID NO:33); or

b) peptides DPI-21.2 (SEQ ID NO:27), DFI-22.2 (SEQ ID NO:28), DPI-23.31 (SEQ ID NO:29), DPI-26.6 (SEQ ID NO:30), DPII-20.9 (SEQ ID NO:31), DPII-22.14 (SEQ ID NO:32) and DPII-25.15 (SEQ ID NO:33).

**8.** An optimised multipeptide formulation suitable for therapeutic treatment of humans suffering from allergy to house dust mite comprising:

a) peptides DPI-21.2 (SEQ ID NO:27), DFI-22.2 (SEQ ID NO:28), DPI-23.31 (SEQ ID NO:29), DPI-26.6 (SEQ ID NO:30), DPII-22.14 (SEQ ID NO:32) and DPII-25.15 (SEQ ID NO:33); and optionally DPII-20.9 (SEQ ID N0:31);

each peptide being in a concentration of 0.75 mg per peptide;

0.05M sodium phosphate U.S.P.;

5% w/v mannitol U.S.P.;

0.1 mg/ml EDTA disodium dehydrate U.S.P.; and

sterile water for injection U.S.P.;

wherein said formulation has a final pH of 7.2-7.4; or

b) peptides DFI-22.2 (SEQ ID NO:28); DPI-23.1 (SEQ ID NO: 29) and DPII-22.14 (SEQ ID NO:32);

each peptide being in a concentration of 0.75 mg per peptide;

0.05M sodium phosphate U.S.P.;

5% w/v mannitol U.S.P.;

0.1 mg/ml EDTA disodium dehydrate U.S.P.; and

sterile water for injection U.S.P.;

wherein said formulation has a final pH of 7.0; or

c) peptides DPI-21.2 (SEQ ID NO:27), DPI-26.6 (SEQ ID NO:30), and DPII-25.15 (SEQ ID NO:33);

each peptide being in a concentration of 0.75 mg per peptide;

0.05M sodium phosphate U.S.P.;

5% w/v mannitol U.S.P.;

0.1 mg/ml EDTA disodium dehydrate U.S.P.; and

sterile water for injection U.S.P.;

wherein said formulation has a final pH of 6.2.

9.  The formulation of claim 8 for use in therapy.

10. First and second optimised multipeptide formulations for simultaneous or sequential use in therapy, wherein said first formulation comprises:

    peptides DFI-22.2 (SEQ ID NO:28); DPI-23.1 (SEQ ID NO: 29) and DPII-22.14 (SEQ ID NO:32);
    each peptide being in a concentration of 0.75 mg per peptide;
    0.05M sodium phosphate U.S.P.;
    5% w/v mannitol U.S.P.;
    0.1 mg/ml EDTA disodium dehydrate U.S.P.; and
    sterile water for injection U.S.P.;
    having a final pH of 7.0;

    and wherein said second optimised multipeptide formulation comprises:

    peptides DPI-21.2 (SEQ ID NO:27), DPI-26.6 (SEQ ID NO:30), and DPII-25.15 (SEQ ID NO:33);
    each peptide being in a concentration of 0.75 mg per peptide;
    0.05M sodium phosphate U.S.P.;
    5% w/v mannitol U.S.P.;
    0.1 mg/ml EDTA disodium dehydrate U.S.P.; and
    sterile water for injection U.S.P.;
    having a final pH of 6.2.

11. A formulation as claimed in claim 9 or claim 10 for use in treating sensitivity to house dust mite allergen.

12. The use of a formulation as claimed in any one of claims 1, 2 or 5 to 8 in the manufacture of a medicament for treatment of sensitivity to house dust mite allergen.

13. An isolated peptide having an amino acid sequence selected from the group consisting of DPI-23.31 (SEQ ID NO: 29), DPI-26.6 (SEQ ID NO:30), DPII-20.9 (SEQ ID NO:31), DPII-22.14 (SEQ ID NO:32) and DPII-25.15 (SEQ ID NO:33).

**Patentansprüche**

1.  Therapeutische Zusammensetzung, die zumindest ein isoliertes Peptid, ausgewählt aus der Gruppe, die aus DPI-21,2 (SEQ ID NO: 27) und DFI-22,2 (SEQ ID NO: 28) besteht und zumindest ein Peptid umfasst, ausgewählt aus der Gruppe, die aus DPI-23,31 (SEQ ID NO: 29), DPI-26,6 (SEQ ID NO: 30), DPII-20,9 (SEQ ID NO: 31), DPII-22,14 (SEQ ID NO: 32) und DPII-25,15 (SEQ ID NO: 33) besteht, die alle in Figur 1 dargestellt sind.

2.  Therapeutische Zusammensetzung, die folgendes umfasst:

    a) die Peptide DFI-22,2 (SEQ ID NO: 28), DPI-23,1 (SEQ ID NO: 29) und DPII-22,14 (SEQ ID NO: 32);
    b) die Peptide DPI-21,2 (SEQ ID NO: 27), DPI-26,6 (SEQ ID NO: 30), und DPII-25,15 (SEQ ID NO: 33);
    c) die Peptide DPI-21,2 (SEQ ID NO: 27), DPI-22,2 (SEQ ID NO: 28), DPI-23,31 (SEQ ID NO: 29), DPI-26,6 (SEQ ID NO: 30), DPII-22,14 (SEQ ID NO: 32) und DPII-25,15 (SEQ ID NO: 33); oder
    d) Peptide DPI-21,2 (SEQ ID NO: 27), DFI-22,2 (SEQ ID NO: 28), DPI-23,31 (SEQ ID NO: 29), DPI-26,6 (SEQ ID NO: 30), DPII-20,9 (SEQ ID NO: 31), DPII-22,14 (SEQ ID NO: 32) und DPII-25,15 (SEQ ID NO: 33).

3.  Zusammensetzung nach Anspruch 1 oder 2 zur Verwendung in der Therapie.

4.  Zusammensetzung nach Anspruch 1 oder Anspruch 2 zur Verwendung in:

    a) einem Verfahren zur Behandlung der Sensibilität gegenüber einem Hausstaubmilbenallergen bei einem Säugetier, umfassend eine gleichzeitige oder aufeinanderfolgende Verabreichung zumindest zwei unterschiedlicher Zusammensetzungen nach Anspruch 1; oder

b) ein Verfahren zur Behandlung der Sensibilität gegenüber einem Hausstaubmilbenallergen in einem Säugetier, umfassend eine Verabreichung einer therapeutischen Zusammensetzung nach Anspruch 2.

5. Multipeptid-Zubereitung zur pharmazeutischen Verabreichung, die folgendes umfasst:

a) zumindest ein isoliertes Peptid, ausgewählt aus der Gruppe, die aus DPI-21,2 (SEQ ID NO: 27) und DFI-22,2 (SEQ ID NO: 28) besteht und zumindest ein Peptid, ausgewählt aus der Gruppe, die aus DPI-23,31 (SEQ ID NO: 29), DPI-26,6 (SEQ ID NO: 30), DPII-20,9 (SEQ ID NO: 31), DPII-22,14 (SEQ ID NO: 32) und DPII-25,15 (SEQ ID NO: 33) besteht, wobei jedes Peptid bei einem pH im Bereich von pH 6,0 bis pH 8,0 löslich und stabil ist und wobei die Zubereitung einen ausreichenden Prozentsatz einer T-Zell-Aktivität von Gruppe I und Gruppe II *Der f*- und Der *p*-Hausstaubmilben-Allergenen umfasst;
b) einen pharmazeutisch verträglichen Träger und wahlweise
c) ein oder mehreres von EDTA und einem pharmazeutisch verträglichen Gegenion.

6. Zubereitung nach Anspruch 5, die zumindest 37% T-Zell-Aktivität von Gruppe 1 und Gruppe II *Der f* und *Der p*-Hausstaubmilben-Allergenen umfasst.

7. Multipeptid-Zubereitung nach Anspruch 5 oder Anspruch 6, die folgendes umfasst:

a) Peptide, die DPI-21,2 (SEQ ID N0: 27), DFI-22,2 (SEQ ID N0: 28), DPI-23,31 (SEQ ID NO: 29), DPI-26,6 (SEQ ID N0: 30), DPII-22,14 (SEQ ID NO: 32) und DPII-25,15 (SEQ ID NO: 33); oder
b) Peptide DPI-21,2 (SEQ ID NO: 27), DFI-22,2 (SEQ ID NO: 28), DPI-23,31 (SEQ ID NO: 29), DPI-26,6 (SEQ ID NO: 30), DPII-20,9 (SEQ ID NO: 31), DPII-22,14 (SEQ ID NO: 32) und DPII-25,15 (SEQ ID NO: 33).

8. Optimierte Multipeptid-Zubereitung, die für eine therapeutische Behandlung von Menschen geeignet ist, die unter einer Allergie gegenüber Hausstaubmilben leiden, die folgendes umfasst:

a) Peptide DPI-21,2 (SEQ ID NO: 27), DFI-22,2. (SEQ ID NO: 28), DPI-23,31 (SEQ ID NO: 29), DPI-26,6 (SEQ ID NO: 30), DPII-29,14 (SEQ ID NO: 32) und DPII-25,15 (SEQ ID NO: 33); und wahlweise DPII-20,9 (SEQ ID No: 31)
wobei jedes Peptid in einer Konzentration von 0,75 mg pro Peptid vorliegt;
0,05M Natriumphosphat U.S.P.;
5% g/v Mannitol U.S.P.;
0,1 mg/ml Dinatrium-EDTA-Dihydrat U.S.P.; und
steriles Wasser zur Injektion U.S.P.; wobei die Formulierung einen End-pH von 7,2-7,4 aufweist; oder
b) Peptide DFI-22,2 (SEQ ID NO: 28), DPI-23,1 (SEQ ID NO: 29) und DPII-22,14 (SEQ ID NO: 32);
wobei jedes Peptid in einer Konzentration von 0,75 mg pro Peptid vorliegt;
0,05M Natriumphosphat U.S.P.;
5% g/v Mannitol U.S.P.;
0,1 mg/ml Dinatrium-EDTA-Dihydrat U.S.P.; und
steriles Wasser zur Injektion U.S.P.; wobei die Zubereitung einen endgültigen pH von 7,0 aufweist; oder
c) Peptide DPI-21,2 (SEQ ID N0: 27), DPI-26,6 (SEQ ID NO: 30), und DPII-25,15 (SEQ ID NO: 33);
wobei jedes Peptid in einer Konzentration von 0,75 mg pro Peptid vorliegt;
0,05M Natriumphosphat U.S.P.;
5% g/v Mannitol U.S.P.;
0,1 mg/ml Dinatrium-EDTA-Dihydrat U.S.P.; und
steriles Wasser zur Injektion U.S.P.; wobei die Zubereitung einen endgültigen pH von 6,2 aufweist.

9. Formulierung nach Anspruch 8 zur Verwendung in der Therapie.

10. Erste und zweite optimierte Multipeptid-Zubereitungen zur simultanen oder sequentiellen Verwendung in der Therapie, wobei die erste Zubereitung folgendes umfasst:

Peptide DFI-22,2 (SEQ ID NO: 28), DPI-23,1 (SEQ ID NO: 29) und DPII-22,14 (SEQ ID N0: 32);
wobei jedes Peptid in einer Konzentration von 0,75 mg pro Peptid vorliegt;
0,05M Natriumphosphat U.S.P.;

5% g/v Mannitol U.S.P.;
0,1 mg/ml Dinatrium-EDTA-Dihydrat U.S.P.; und
steriles Wasser zur Injektion U.S.P.;
mit einem endgültigen pH von 7,0;

und wobei die zweite optimierte Multipeptid-Zubereitung folgendes umfasst:

Peptide DPI-21,2 (SEQ ID NO: 27), DPI-26,6 (SEQ ID NO: 30) und DPII-25,15 (SEQ ID NO: 33);
wobei jedes Peptid in einer Konzentration von 0,75 mg pro Peptid vorliegt;
0,05M Natriumphosphat U.S.P.;
5% g/v Mannitol U.S.P.;
0,1 mg/ml Dinatrium-EDTA Dihydrat U.S.P.; und
steriles Wasser zur Injektion U.S.P.;
mit einem endgültigen pH von 6,2.

**11.** Zubereitung nach Anspruch 9 oder Anspruch 10 zur Verwendung in der Behandlung einer Sensibilität gegenüber einem Hausstaubmilben-Allergen.

**12.** Verwendung einer Zubereitung nach einem der Ansprüche 1, 2 oder 5 bis 8 in der Herstellung eines Medikamentes zur Behandlung der Sensibilität gegenüber einem Hausstaubmilben-Allergen.

**13.** Isoliertes Peptid mit einer Aminosäuresequenz ausgewählt aus der Gruppe, die aus DPI-23,31 (SEQ ID NO: 29), DPI-26,6 (SEQ ID NO: 30), DPII-20,9 (SEQ ID NO: 31), DPII-22,14 (SEQ ID NO: 32) und DPII-25,15 (SEQ ID NO: 33) besteht.

**Revendications**

**1.** Composition thérapeutique comprenant au moins un peptide isolé choisi parmi le groupe constitué des peptides DPI-21.2 (ID SEQ No.27), et DFI-22.2 (ID SEQ No.28), et au moins un peptide choisi parmi le groupe constitué des peptides DPI-23-31 (ID SEQ No.29), DPI-26.6 (ID SEQ No.30), DPII-20.9 (ID SEQ No.31), DPII-22.14 (ID SEQ No. 32), et DPII-25.15 (ID SEQ No.33) tels que représentés à la figure 1.

**2.** Composition thérapeutique comprenant :

a) les peptides DFI-22.2 (ID SEQ No.28), DPI-23.1 (ID SEQ No.29), et DPII-22.14 (ID SEQ No.32);
b) les peptides DPI-21.2 (ID SEQ No.27), DPI-26.6 (ID SEQ No.30), et DPII-25.15 (ID SEQ No.33);
c) les peptides DPI-21-2 (ID SEQ No.27), DFI-22.2 (ID SEQ No.28), DPI-23-31 (ID SEQ No.29), DPI-26.6 (ID SEQ No.30), et DPI-22.14 (ID SEQ No.32), et DPII-25-15 (ID SEQ No.33) ; ou
d) les peptides DPI-21.2 (ID SEQ No.27), DFI-22.2 (ID SEQ No.28), DPI-23.31 (ID SEQ No.29), DPI-26.6 (ID SEQ No.30), DPII-20.9 (ID SEQ No.31), DPII-22.14 (ID SEQ No.32), et DPII-25.15 (ID SEQ No.33).

**3.** Composition selon la revendication 1 ou 2 pour usage thérapeutique.

**4.** Composition selon la revendication 1 ou 2 pour usage :

a) comme traitement des réactions à l'allergène acarien détriticole chez les mammifères comprenant une administration simultanée ou consécutive d'au moins deux compositions différentes selon la revendication 1; ou
b) comme traitement des réactions à l'allergène acarien détriticole chez les mammifères comprenant l'administration d'une composition thérapeutique selon la revendication 2.

**5.** Formulation multipeptide pour administration pharmaceutique comprenant :

a) au moins un peptide isolé choisi parmi le groupe constitué des peptides DPI-21.2 (ID SEQ No.27), et DFI-22.2 (ID SEQ No.28), et au moins une peptide choisi parmi le groupe constitué des peptides DPI-23.31 (ID SEQ No.29), DPI-26.6 (ID SEQ No.30), DPII-20.9 (ID SEQ No.31), DPII-22.14 (ID SEQ No.32), et DPII-25.15 (ID SEQ No.33), chaque peptide étant soluble et stable à un pH compris entre pH 6,0 et pH 8,0, et où la dite formulation comprend un pourcentage suffisant d'activité des cellules T du Groupe I et du Groupe II *Der f* et *Der p* d'allergènes acarien détriticole ;

b) un excipient acceptable au niveau pharmaceutique, et éventuellement,

c) un ou plus EDTA et un contre-ion acceptable au niveau pharmaceutique.

**6.** Formulation selon la revendication 5, qui comprend au moins 37 % d'activité des cellules T du Groupe 1 ou du Groupe II *Der f* et *Der p* d'allergènes acarien détriticole.

**7.** Formulation selon la revendication 5 ou 6 comprenant:

    a) les peptides DPI-21.2 (ID SEQ No.27), DFI-22.2 (ID SEQ No. 28), DPI-23.31 (ID SEQ No. 29), DPI-26.6 (ID SEQ No. 30), DPII-22.14 (ID SEQ No. 32), et DPII-25.15 (ID SEQ No. 33); ou
    b) les peptides DFI-21.2 (ID SEQ No.27), DFI-22.2 (ID SEQ No. 28), DPI-23.31 (ID SEQ No. 29), DPI-26.6 (ID SEQ No. 30), DPII-20.9 (ID SEQ No. 31), DPII-22.14 (ID SEQ No. 32), et DPII-25.15 (ID SEQ No.33).

**8.** Formulation multipeptide optimisée qui convient au traitement thérapeutique des humains souffrant d'allergies à l'acarien détriticole comprenant :

    a) les peptides DPI-21.2 (ID SEQ No. 27), DFI-22.2 (ID SEQ No. 28), DPI-23-31 (ID SEQ No. 29), DPI-26.6 (ID SEQ No. 30), DPII-22.14 ((ID SEQ No. 32), et DPII-25.15 (ID SEQ No. 33) et, éventuellement DPII-20.9 (ID SEQ No. 31)
    chaque peptide étant sous concentration de 0,75 mg par peptide;
    0,05 M de phosphate de sodium USP ;
    5 % pds/v de mannitol USP ;
    0,1 mg/ml d'EDTA disodium dihydrate USP ; et de l'eau stérilisée pour injection USP ; où ladite formulation dispose d'un pH final compris entre 7,2 et 7,4 ; ou
    b) les peptides DFI-22.2 (ID SEQ No. 28), DPI-23.1 (ID SEQ No. 29), et DPII-22.14 ((ID SEQ No. 32);
    chaque peptide étant sous concentration de 0,75 mg par peptide ;
    0,05 M de phosphate de sodium USP;
    5 % pds/v de mannitol USP ;
    0,1 mg/ml d'EDTA disodium dihydrate USP ; et de l'eau stérilisée pour injection USP ; où ladite formulation dispose d'un pH final égal à 7,0; ou
    c) les peptides DPI-21.2 (ID SEQ No. 27), DPI-26.6 (ID SEQ No. 30), et DPII-25.15 (ID SEQ No. 33);
    chaque peptide étant sous concentration de 0,75 mg par peptide;
    0,05 M de phosphate de sodium USP;
    5 % pds/v de mannitol USP;
    0,1 mg/ml d'EDTA disodium dihydrate USP ; et de l'eau stérilisée pour injection USP; où ladite formulation dispose d'un pH final égal à 6,2.

**9.** Formulation selon la revendication 8 pour usage thérapeutique.

**10.** La première et la deuxième formulation multipeptide optimisée pour administration thérapeutique simultanée ou consécutive, où la première formulation comprend:

    les peptides DFI-22.2 (ID SEQ No. 28), DPI-23.1 (ID SEQ No. 29), et DPII-21.14 (ID SEQ No. 32);
    chaque peptide étant sous concentration de 0,75 mg par peptide ;
    0,05 M de phosphate de sodium USP;
    5 % pds/v de mannitol USP ;
    0,1 mg/ml d'EDTA disodium dihydrate USP ; et de l'eau stérilisée pour injection USP ; où ladite formulation dispose d'un pH final égal à 7,0;

et où ladite deuxième formulation multipeptide optimisée comprend les peptides DPI-21.2 (ID SEQ No. 27, DPI-26.6 (ID SEQ No. 30), et DPII-25.15 (ID SEQ No. 33);

    chaque peptide étant sous concentration de 0,75 mg par peptide ;
    0,05 M de phosphate de sodium USP ;
    5 % pds/v de mannitol USP ;
    0,1 mg/ml d'EDTA disodium dihydrate USP ; et de l'eau
    stérilisée pour injection USP ; où ladite formulation

dispose d'un pH final égal à 6,2.

11. Formulation selon l'une quelconque des revendications 9 ou 10, destinée au traitement des réactions à l'allergène acarien détriticole.

12. Utilisation de la formulation selon l'une quelconque des revendications 1, 2 ou 5 à 8 pour la fabrication d'un médicament conçu pour le traitement des réactions à l'allergène acarien détriticole.

13. Peptide isolé dont la séquence d'acides aminés est choisie parmi le groupe constitué de DPI-23.31 (ID SEQ No. 29), DPI-23.31 (ID SEQ No. 29), DPI-26.6 (ID SEQ No. 30), DPII-20.9 (ID SEQ No. 31), DPII-22.14 (ID SEQ No. 32), et DPII-25.15 (ID SEQ No. 33).

## Fig. 1

| NAME | SEQUENCE |
|---|---|
| DPI-21.2 | S I N G N A P A E I D L R Q M R T V T P I R M Q |
| DFI-22.2 | V A A T E S A Y L A Y R N T S L D L S E Q E L V D |
| DPI-23.31 | D E E G V V Q E S Y Y R Y V A R E Q S C R R P N A E |
| DPI-26.6 | D E G I K D L D A F R H Y D G R T I I Q R D N G Y E |
| DPII-20.9 | D Q V D V K D C A N H E I K K V L V P G C H G S E |
| DPII-22.14 | D K E L E A V F E A N Q N T K T A K A E |
| DPII-25.15 | D E E T V K V M G D D G V L A C A I A T H A K I R D E E |

# Fig. 2

Peptide Name

| | |
|---|---|
| DPI-1 (1-20) | T N A C S I N G N A P A E I D L R Q M R |
| DPI-2 (13-39) | E I D L R Q M R T V T P I R M Q G G C G S C W A F S G |
| DPI-3 (21-49) | T V T P I R M Q G G C G S C W A F S G V A A T E S A Y L A |
| DPI-4 (40-60) | V A A T E S A Y L A H R N Q S L D L A E Q |
| DPI-11.1 (50-71) | H R N Q S V D L A E Q E L V D C A S Q H G C |
| DPI-12.1 (61-81) | E L V D C A S Q H G C H G D T I P R G I E |
| DPI-5.1 (81-100) | E Y I Q H N G V V Q E S Y Y R Y V A R E |
| DIP-13 (85-109) | H N G V V Q E S Y Y R Y V A R E Q S C R R P N A Q |
| DPI-14 (101-119) | Q S C R R P N A Q R F G I S N Y C Q I |
| DPI-15 (110-131) | R F G I S N Y C Q I Y P P N A N K I R E A L |
| DPI-6.1 (120-143) | Y P P N A N K I R E A L A Q T H S A I A V I I G |
| DPI-7.1 (132-157) | A Q T H S A I A V I I G I K D L D A F R H Y D G R T |
| DPI-8 (144-169) | I K D L D A F R H Y D G R T I I Q R D N G Y Q P N Y |
| DPI-9 (158-180) | I I Q R D N G Y Q P N Y H A V N I V G Y S N A |
| DPI-16 (170-191) | H A V N I V G Y S N A Q G V D Y W I V R N S |
| DPI-10 (181-204) | Q G V D Y W I V R N S W D T N W G D N G Y G Y F |
| DPI-17 (197-222) | G D N G Y G Y F A A N I D L M M I E E Y P Y V V I L |
| DPII-1 (1-20) | D Q V D V K D C A N H E I K K V L V P G |
| DPII-2 (11-35) | H E I K K V L V P G C H G S E P C I I H R G K P F |
| DPII-3.1 (22-50) | H G S E P C I I H R G K P F Q L E A V F E A N Q N T K T A |
| DPII-4 (36-60) | Q L E A V F E A N Q N T K T A K I E I K A S I D G |
| DPII-5 (51-77) | K I E I K A S I D G L E V D V P G I D P N A C H Y M K |
| DPII-6 (61-86) | L E V D V P G I D P N A C H Y M K C P L V K G Q Q Y |
| DPII-7 (78-104) | C P L V K G Q Q Y D I K Y T W N V P K I A P K S E N V |
| DPII-8 (87-112) | D I K Y T W N V P K I A P K S E N V V V T V K V M G |
| DPII-9 (105-129) | V V T V K V M G D D G V L A C A I A T H A K I R D |

## *Fig. 3*

N=39

Fig. 4

# *Fig. 5*

## Fig. 6

Legend:
- 3 mg/mL at 25°C
- 2 mg/mL at 25°C
- 1 mg/mL at 25°C
- 3 mg/mL at 5°C
- 2 mg/mL at 5°C
- 1 mg/mL at 5°C

Y-axis: % Degradation (Peak Area)
X-axis: pH

SEQUENCE LISTING

(1) GENERAL INFORMATION:

(1) GENERAL INFORMATION:

(i) APPLICANT:

(A) NAME: IMMULOGIC PHARMACEUTICAL CORPORATION
(B) STREET: 610 Lincoln Street
(C) CITY: Waltham
(D) STATE: MA
(E) COUNTRY: USA
(F) POSTAL CODE (ZIP): 02154
(G) TELEPHONE: (617) 466-6000
(H) TELEFAX: (617)466-6040

(ii) TITLE OF INVENTION: PHARMACEUTICAL PEPTIDE FORMULATIONS FOR TREATMENT OF DUST MITE ALLERGY

(iii) NUMBER OF SEQUENCES: 54

(iv) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(vi) PRIOR APPLICATION DATA:

(A) APPLICATION NUMBER: 08/227,772
(B) FILING DATE: April 14, 1994

(viii) ATTORNEY/AGENT INFORMATION:

(A) NAME: Vanstone, Darlene A.

(B) REGISTRATION NUMBER: 35,279
(C) REFERENCE/DOCKET NUMBER: 017.5 PCT

(ix) TELECOMMUNICATION INFORMATION:

(A) TELEPHONE: (617) 466-6000
(B) TELEFAX: (617) 466-6010

(2) INFORMATION FOR SEQ ID NO: I:

(i) SEQUENCE CHARACTERISTICS:

(A) LENGTH: 20 amino acids
(B) TYPE: amino acid
(C) STRANDEDNESS:
(D) TOPOLOGY: linear

(ii) MOLECULE TYPE: peptide

(v) FRAGMENT TYPE: internal

(xi) SEQUENCE DESCRIPTION: SEQ ID NO: 1:

```
Thr Asn Ala Cys Ser Ile Asn Gly Asn Ala Pro Ala Glu Ile Asp
1               5                   10                  15


Leu Arg Gln Met Arg                    .
                20
```

(2) INFORMATION FOR SEQ ID NO: 2:

(i) SEQUENCE CHARACTERISTICS:

(A) LENGTH: 27 amino acids
(B) TYPE: amino acid
(C) STRANDEDNESS:
(D) TOPOLOGY: linear

(ii) MOLECULE TYPE: peptide
(v) FRAGMENT TYPE: internal
(xi) SEQUENCE DESCRIPTION: SEQ ID NO: 2:

```
Glu Ile Asp Leu Arg Gln Met Arg Thr Val Thr Pro Ile Arg Met Gln
1               5                   10                  15

Gly Gly Cys Gly Ser Cys Trp Ala Phe Ser Gly
            20              25
```

(2) INFORMATION FOR SEQ ID NO: 3:

(i) SEQUENCE CHARACTERISTICS:

(A) LENGTH: 29 amino acids
(B) TYPE: amino acid

(C) STRANDEDNESS:
(D) TOPOLOGY: linear

(ii) MOLECULE TYPE: peptide
(v) FRAGMENT TYPE: internal
(xi) SEQUENCE DESCRIPTION: SEQ ID NO: 3:

```
Thr Val Thr Pro Ile Arg Met Gln Gly Gly Cys Gly Ser Cys Trp Ala
1               5               10              15

Phe Ser Gly Val Ala Ala Thr Glu Ser Ala Tyr Leu Ala
            20              25
```

(2) INFORMATION FOR SEQ ID NO: 4:

(i) SEQUENCE CHARACTERISTICS:

(A) LENGTH: 21 amino acids
(B) TYPE: amino acid
(C) STRANDEDNESS:
(D) TOPOLOGY: linear

(ii) MOLECULE TYPE: peptide
(v) FRAGMENT TYPE: internal
(xi) SEQUENCE DESCRIPTION: SEQ ID NO: 4:

```
Val Ala Ala Thr Glu Ser Ala Tyr Leu Ala His Arg Asn Gln Ser Leu

1               5               10              15

Asp Leu Ala Glu Gln
            20
```

(2) INFORMATION FOR SEQ ID NO: 5:

(i) SEQUENCE CHARACTERISTICS:

(A) LENGTH: 22 amino acids
(B) TYPE: amino acid
(C) STRANDEDNESS:
(D) TOPOLOGY: linear

(ii) MOLECULE TYPE: peptide
(v) FRAGMENT TYPE: internal
(xi) SEQUENCE DESCRIPTION: SEQ ID NO: 5:

```
His Arg Asn Gln Ser Val Asp Leu Ala Glu Gln Glu Leu Val Asp Cys
1               5               10              15

Ala Ser Gln His Gly Cys
            20
```

(2) INFORMATION FOR SEQ ID NO: 6:

(i) SEQUENCE CHARACTERISTICS:

    (A) LENGTH: 21 amino acids
    (B) TYPE: amino acid
    (C) STRANDEDNESS:
    (D) TOPOLOGY: linear

(ii) MOLECULE TYPE: peptide
(v) FRAGMENT TYPE: internal
(xi) SEQUENCE DESCRIPTION: SEQ ID NO: 6:

```
Glu Leu Val Asp Cys Ala Ser Gln His Gly Cys His Gly Asp Thr Ile
1               5                   10                  15

Pro Arg Gly Ile Glu
            20
```

(2) INFORMATION FOR SEQ ID NO: 7:

(i) SEQUENCE CHARACTERISTICS:

    (A) LENGTH: 20 amino acids
    (B) TYPE: amino acid
    (C) STRANDEDNESS:
    (D) TOPOLOGY: linear

(ii) MOLECULE TYPE: peptide
(v) FRAGMENT TYPE: internal
(xi) SEQUENCE DESCRIPTION: SEQ ID NO: 7:

```
Glu Tyr Ile Gln His Asn Gly Val Val Gln Glu Ser Tyr Tyr Arg Tyr
1               5                   10                  15

Val Ala Arg Glu
            20
```

(2) INFORMATION FOR SEQ ID NO: 8:

(i) SEQUENCE CHARACTERISTICS:

    (A) LENGTH: 25 amino acids
    (B) TYPE: amino acid
    (C) STRANDEDNESS:
    (D) TOPOLOGY: linear

(ii) MOLECULE TYPE: peptide
(v) FRAGMENT TYPE: internal
(xi) SEQUENCE DESCRIPTION: SEQ ID NO: 8:

```
His Asn Gly Val Val Gln Glu Ser Tyr Tyr Arg Tyr Val Ala Arg Glu
1               5                   10                  15

Gln Ser Cys Arg Arg Pro Asn Ala Gln
            20                  25
```

(2) INFORMATION FOR SEQ ID NO: 9:

    (i) SEQUENCE CHARACTERISTICS:

        (A) LENGTH: 19 amino acids
        (B) TYPE: amino acid
        (C) STRANDEDNESS:
        (D) TOPOLOGY: linear

    (ii) MOLECULE TYPE: peptide
    (v) FRAGMENT TYPE: internal
    (xi) SEQUENCE DESCRIPTION: SEQ ID NO: 9:

```
Gln Ser Cys Arg Arg Pro Asn Ala Gln Arg Phe Gly Ile Ser Asn Tyr
1               5                   10                  15

Cys Gln Ile
```

(2) INFORMATION FOR SEQ ID NO: 10:

    (i) SEQUENCE CHARACTERISTICS:

        (A) LENGTH: 22 amino acids
        (B) TYPE: amino acid
        (C) STRANDEDNESS:
        (D) TOPOLOGY: linear

    (ii) MOLECULE TYPE: peptide
    (v) FRAGMENT TYPE: internal
    (xi) SEQUENCE DESCRIPTION: SEQ ID NO: 10:

```
Arg Phe Gly Ile Ser Asn Tyr Cys Gln Ile Tyr Pro Pro Asn Ala Asn
1               5                   10                  15

Lys Ile Arg Glu Ala Leu
            20
```

(2) INFORMATION FOR SEQ ID NO: 11:

    (i) SEQUENCE CHARACTERISTICS:

        (A) LENGTH: 24 amino acids
        (B) TYPE: amino acid
        (C) STRANDEDNESS:
        (D) TOPOLOGY: linear

    (ii) MOLECULE TYPE: peptide
    (v) FRAGMENT TYPE: internal
    (xi) SEQUENCE DESCRIPTION: SEQ ID NO: 11:

```
Tyr Pro Pro Asn Ala Asn Lys Ile Arg Glu Ala Leu Ala Gln Thr His
1               5                   10                  15

Ser Ala Ile Ala Val Ile Ile Gly
                20
```

(2) INFORMATION FOR SEQ ID NO: 12:

    (i) SEQUENCE CHARACTERISTICS:

        (A) LENGTH: 26 amino acids
        (B) TYPE: amino acid
        (C) STRANDEDNESS:
        (D) TOPOLOGY: linear

    (ii) MOLECULE TYPE: peptide
    (v) FRAGMENT TYPE: internal
    (xi) SEQUENCE DESCRIPTION: SEQ ID NO: 12:

```
Ala Gln Thr His Ser Ala Ile Ala Val Ile Ile Gly Ile Lys Asp Leu
1               5                   10                  15

Asp Ala Phe Arg His Tyr Asp Gly Arg Thr
                20                  25
```

(2) INFORMATION FOR SEQ ID NO: 13:

    (i) SEQUENCE CHARACTERISTICS:

        (A) LENGTH: 26 amino acids
        (B) TYPE: amino acid
        (C) STRANDEDNESS:
        (D) TOPOLOGY: linear

    (ii) MOLECULE TYPE: peptide
    (v) FRAGMENT TYPE: internal
    (xi) SEQUENCE DESCRIPTION: SEQ ID NO: 13:

```
Ile Lys Asp Leu Asp Ala Phe Arg His Tyr Asp Gly Arg Thr Ile Ile
1               5                   10                  15

Gln Arg Asp Asn Gly Tyr Gln Pro Asn Tyr
                20                  25
```

(2) INFORMATION FOR SEQ ID NO: 14:

    (i) SEQUENCE CHARACTERISTICS:

        (A) LENGTH: 23 amino acids
        (B) TYPE: amino acid
        (C) STRANDEDNESS:
        (D) TOPOLOGY: linear

    (ii) MOLECULE TYPE: peptide
    (v) FRAGMENT TYPE: internal

(xi) SEQUENCE DESCRIPTION: SEQ ID NO: 14:

```
        Ile Ile Gln Arg Asp Asn Gly Tyr Gln Pro Asn Tyr His Ala Val Asn
        1               5                   10              15

        Ile Val Gly Tyr Ser Asn Ala
                    20
```

(2) INFORMATION FOR SEQ ID NO: 15:

(i) SEQUENCE CHARACTERISTICS:

(A) LENGTH: 22 amino acids
(B) TYPE: amino acid
(C) STRANDEDNESS:
(D) TOPOLOGY: linear

(ii) MOLECULE TYPE: peptide
(v) FRAGMENT TYPE: internal
(xi) SEQUENCE DESCRIPTION: SEQ ID NO: 15:

```
        His Ala Val Asn Ile Val Gly Tyr Ser Asn Ala Gln Gly Val Asp Tyr
        1               5                   10              15

        Trp Ile Val Arg Asn Ser
                    20
```

(2) INFORMATION FOR SEQ ID NO: 16:

(i) SEQUENCE CHARACTERISTICS:

(A) LENGTH: 24 amino acids
(B) TYPE: amino acid
(C) STRANDEDNESS:
(D) TOPOLOGY: linear

(ii) MOLECULE TYPE: peptide
(v) FRAGMENT TYPE: internal
(xi) SEQUENCE DESCRIPTION: SEQ ID NO: 16:

```
        Gln Gly Val Asp Tyr Trp Ile Val Arg Asn Ser Trp Asp Thr Asn Trp
        1               5                   10              15

        Gly Asp Asn Gly Tyr Gly Tyr Phe
                    20
```

(2) INFORMATION FOR SEQ ID NO: 17:

(i) SEQUENCE CHARACTERISTICS:

(A) LENGTH: 26 amino acids
(B) TYPE: amino acid
(C) STRANDEDNESS:
(D) TOPOLOGY: linear

(ii) MOLECULE TYPE: peptide

(v) FRAGMENT TYPE: internal
(xi) SEQUENCE DESCRIPTION: SEQ ID NO: 17:

```
Gly Asp Asn Gly Tyr Gly Tyr Phe Ala Ala Asn Ile Asp Leu Met Met
1                5                10                15

Ile Glu Glu Tyr Pro Tyr Val Val Ile Leu
        20                25
```

(2) INFORMATION FOR SEQ ID NO: 18:

(i) SEQUENCE CHARACTERISTICS:

(A) LENGTH: 20 amino acids
(B) TYPE: amino acid
(C) STRANDEDNESS:
(D) TOPOLOGY: linear

(ii) MOLECULE TYPE: peptide
(v) FRAGMENT TYPE: internal
(xi) SEQUENCE DESCRIPTION: SEQ ID NO: 18:

```
Asp Gln Val Asp Val Lys Asp Cys Ala Asn His Glu Ile Lys Lys Val
1                5                10                15

Leu Val Pro Gly
        20
```

(2) INFORMATION FOR SEQ ID NO: 19:

(i) SEQUENCE CHARACTERISTICS:

(A) LENGTH: 25 amino acids
(B) TYPE: amino acid
(C) STRANDEDNESS:
(D) TOPOLOGY: linear

(ii) MOLECULE TYPE: peptide
(v) FRAGMENT TYPE: internal
(xi) SEQUENCE DESCRIPTION: SEQ ID NO: 19:

```
His Glu Ile Lys Lys Val Leu Val Pro Gly Cys His Gly Ser Glu Pro
1                5                10                15

Cys Ile Ile His Arg Gly Lys Pro Phe
        20                25
```

(2) INFORMATION FOR SEQ ID NO: 20:

(i) SEQUENCE CHARACTERISTICS:

(A) LENGTH: 29 amino acids
(B) TYPE: amino acid
(C) STRANDEDNESS:
(D) TOPOLOGY: linear

(ii) MOLECULE TYPE: peptide

(v) FRAGMENT TYPE: internal

(xi) SEQUENCE DESCRIPTION: SEQ ID NO: 20:


```
His Gly Ser Glu Pro Cys Ile Ile His Arg Gly Lys Pro Phe Gln Leu
1               5               10              15

Glu Ala Val Phe Glu Ala Asn Gln Asn Thr Lys Thr Ala
            20              25
```

(2) INFORMATION FOR SEQ ID NO: 21:

(i) SEQUENCE CHARACTERISTICS:

(A) LENGTH: 25 amino acids

(B) TYPE: amino acid

(C) STRANDEDNESS:

(D) TOPOLOGY: linear

(ii) MOLECULE TYPE: peptide

(v) FRAGMENT TYPE: internal

(xi) SEQUENCE DESCRIPTION: SEQ ID NO: 21:


```
Gln Leu Glu Ala Val Phe Glu Ala Asn Gln Asn Thr Lys Thr Ala Lys
1               5               10              15

Ile Glu Ile Lys Ala Ser Ile Asp Gly
            20              25
```

(2) INFORMATION FOR SEQ ID NO: 22:

(i) SEQUENCE CHARACTERISTICS:

(A) LENGTH: 27 amino acids

(B) TYPE: amino acid

(C) STRANDEDNESS:

(D) TOPOLOGY: linear

(ii) MOLECULE TYPE: peptide

(xi) SEQUENCE DESCRIPTION: SEQ ID NO: 22:


```
Lys Ile Glu Ile Lys Ala Ser Ile Asp Gly Leu Glu Val Asp Val Pro
1               5               10              15

Gly Ile Asp Pro Asn Ala Cys His Tyr Met Lys
            20              25
```

(2) INFORMATION FOR SEQ ID NO: 23:

(i) SEQUENCE CHARACTERISTICS:

(A) LENGTH: 26 amino acids

(B) TYPE: amino acid

(C) STRANDEDNESS:

(D) TOPOLOGY: linear

(ii) MOLECULE TYPE: peptide
(v) FRAGMENT TYPE: internal
(xi) SEQUENCE DESCRIPTION: SEQ ID NO: 23:

```
Leu Glu Val Asp Val Pro Gly Ile Asp Pro Asn Ala Cys His Tyr Met
1               5               10              15

Lys Cys Pro Leu Val Lys Gly Gln Gln Tyr
            20              25
```

(2) INFORMATION FOR SEQ ID NO: 24:

(i) SEQUENCE CHARACTERISTICS:

(A) LENGTH: 27 amino acids
(B) TYPE: amino acid
(C) STRANDEDNESS:
(D) TOPOLOGY: linear

(ii) MOLECULE TYPE: peptide
(v) FRAGMENT TYPE: internal
(xi) SEQUENCE DESCRIPTION: SEQ ID NO: 24:

```
Cys Pro Leu Val Lys Gly Gln Gln Tyr Asp Ile Lys Tyr Thr Trp Asn
1               5               10              15

Val Pro Lys Ile Ala Pro Lys Ser Glu Asn Val
            20              25
```

(2) INFORMATION FOR SEQ ID NO: 25:

(i) SEQUENCE CHARACTERISTICS:

(A) LENGTH: 26 amino acids
(B) TYPE: amino acid
(C) STRANDEDNESS:
(D) TOPOLOGY: linear

(ii) MOLECULE TYPE: peptide
(v) FRAGMENT TYPE: internal
(xi) SEQUENCE DESCRIPTION: SEQ ID NO: 25:

```
Asp Ile Lys Tyr Thr Trp Asn Val Pro Lys Ile Ala Pro Lys Ser Glu
1               5               10              15

Asn Val Val Val Thr Val Lys Val Met Gly
            20              25
```

(2) INFORMATION FOR SEQ ID NO: 26:

(i) SEQUENCE CHARACTERISTICS:

(A) LENGTH: 25 amino acids
(B) TYPE: amino acid
(C) STRANDEDNESS :

(D) TOPOLOGY: linear

(ii) MOLECULE TYPE: peptide
(v) FRAGMENT TYPE: internal
(xi) SEQUENCE DESCRIPTION: SEQ ID NO: 26:

```
Val Val Thr Val Lys Val Met Gly Asp Asp Gly Val Leu Ala Cys Ala
1               5               10              15

Ile Ala Thr His Ala Lys Ile Arg Asp
            20              25
```

(2) INFORMATION FOR SEQ ID NO: 27:

(i) SEQUENCE CHARACTERISTICS:

(A) LENGTH: 24 amino acids
(B) TYPE: amino acid
(C) STRANDEDNESS:
(D) TOPOLOGY: linear

(ii) MOLECULE TYPE: peptide
(v) FRAGMENT TYPE: internal
(xi) SEQUENCE DESCRIPTION: SEQ ID NO: 27:

```
Ser Ile Asn Gly Asn Ala Pro Ala Glu Ile Asp Leu Arg Gln Met Arg
1               5               10              15

Thr Val Thr Pro Ile Arg Met Gln
            20
```

(2) INFORMATION FOR SEQ ID NO: 28:

(i) SEQUENCE CHARACTERISTICS:

(A) LENGTH: 25 amino acids
(B) TYPE: amino acid
(C) STRANDEDNESS:
(D) TOPOLOGY: linear

(ii) MOLECULE TYPE: peptide
(v) FRAGMENT TYPE: internal
(xi) SEQUENCE DESCRIPTION: SEQ ID NO: 28:

```
Val Ala Ala Thr Glu Ser Ala Tyr Leu Ala Tyr Arg Asn Thr Ser Leu
1               5               10              15

Asp Leu Ser Glu Gln Glu Leu Val Asp
            20              25
```

(2) INFORMATION FOR SEQ ID NO: 29:

(i) SEQUENCE CHARACTERISTICS:

(A) LENGTH: 26 amino acids

(B) TYPE: amino acid
(C) STRANDEDNESS:
(D) TOPOLOGY: linear

(ii) MOLECULE TYPE: peptide
(v) FRAGMENT TYPE: internal
(xi) SEQUENCE DESCRIPTION: SEQ ID NO: 29:

```
Asp Glu Glu Gly Val Val Gln Glu Ser Tyr Tyr Arg Tyr Val Ala Arg
1               5               10              15

Glu Gln Ser Cys Arg Arg Pro Asn Ala Glu
            20              25
```

(2) INFORMATION FOR SEQ ID NO: 30:

(i) SEQUENCE CHARACTERISTICS:

(A) LENGTH: 26 amino acids
(B) TYPE: amino acid
(C) STRANDEDNESS:
(D) TOPOLOGY: linear

(ii) MOLECULE TYPE: peptide
(v) FRAGMENT TYPE: internal
(xi) SEQUENCE DESCRIPTION: SEQ ID NO: 30:

```
Asp Glu Gly Ile Lys Asp Leu Asp Ala Phe Arg His Tyr Asp Gly Arg
1               5               10              15

Thr Ile Ile Gln Arg Asp Asn Gly Tyr Glu
            20              25
```

(2) INFORMATION FOR SEQ ID NO: 31:

(i) SEQUENCE CHARACTERISTICS:

(A) LENGTH: 25 amino acids
(B) TYPE: amino acid
(C) STRANDEDNESS:
(D) TOPOLOGY: linear

(ii) MOLECULE TYPE: peptide
(xi) SEQUENCE DESCRIPTION: SEQ ID NO: 31:

```
Asp Gln Val Asp Val Lys Asp Cys Ala Asn His Glu Ile Lys Lys Val
1               5               10              15

Leu Val Pro Gly Cys His Gly Ser Glu
            20              25
```

(2) INFORMATION FOR SEQ ID NO: 32:

(i) SEQUENCE CHARACTERISTICS:

(A) LENGTH: 20 amino acids

(B) TYPE: amino acid
(C) STRANDEDNESS:
(D) TOPOLOGY: linear

(ii) MOLECULE TYPE: peptide
(xi) SEQUENCE DESCRIPTION: SEQ ID NO: 32:

```
Asp Lys Glu Leu Glu Ala Val Phe Glu Ala Asn Gln Asn Thr Lys Thr
1               5                   10                  15

Ala Lys Ala Glu
            20
```

(2) INFORMATION FOR SEQ ID NO: 33:

(i) SEQUENCE CHARACTERISTICS:

(A) LENGTH: 28 amino acids
(B) TYPE: amino acid
(C) STRANDEDNESS:
(D) TOPOLOGY: linear

(ii) MOLECULE TYPE: peptide
(v) FRAGMENT TYPE: internal
(xi) SEQUENCE DESCRIPTION: SEQ ID NO: 33:

```
Asp Glu Glu Thr Val Lys Val Met Gly Asp Asp Gly Val Leu Ala Cys
1               5                   10                  15

Ala Ile Ala Thr His Ala Lys Ile Arg Asp Glu Glu
            20                  25
```

(2) INFORMATION FOR SEQ ID NO: 34:

(i) SEQUENCE CHARACTERISTICS:

(A) LENGTH: 28 amino acids
(B) TYPE: amino acid
(C) STRANDEDNESS:
(D) TOPOLOGY: linear

(ii) MOLECULE TYPE: peptide
(v) FRAGMENT TYPE: internal
(xi) SEQUENCE DESCRIPTION: SEQ ID NO: 34:

```
Gln Leu Glu Ala Val Phe Glu Ala Asn Gln Asn Thr Lys Thr Ala Lys
1               5                   10                  15

Ile Glu Ile Lys Ala Ser Ile Asp Gly Leu Glu Val
            20                  25
```

(2) INFORMATION FOR SEQ ID NO: 35:

(i) SEQUENCE CHARACTERISTICS:

(A) LENGTH: 30 amino acids
(B) TYPE: amino acid
(C) STRANDEDNESS:
(D) TOPOLOGY: linear

(ii) MOLECULE TYPE: peptide
(v) FRAGMENT TYPE: internal
(xi) SEQUENCE DESCRIPTION: SEQ ID NO: 35:

```
Lys Gln Leu Glu Ala Val Phe Glu Ala Asn Gln Asn Thr Lys Thr Ala
1               5               10              15

Lys Ile Glu Ile Lys Ala Ser Ile Asp Gly Leu Glu Val Lys
        20              25              30
```

(2) INFORMATION FOR SEQ ID NO: 36:

(i) SEQUENCE CHARACTERISTICS:

(A) LENGTH: 31 amino acids
(B) TYPE: amino acid
(C) STRANDEDNESS:
(D) TOPOLOGY: linear

(ii) MOLECULE TYPE: peptide
(v) FRAGMENT TYPE: internal
(xi) SEQUENCE DESCRIPTION: SEQ ID NO: 36:

```
Asp Lys Gln Leu Glu Ala Val Phe Glu Ala Asn Gln Asn Thr Lys Thr
1               5               10              15

Ala Lys Ile Glu Ile Lys Ala Ser Ile Asp Gly Leu Glu Val Asp
        20              25              30
```

(2) INFORMATION FOR SEQ ID NO: 37:

(i) SEQUENCE CHARACTERISTICS:

(A) LENGTH: 25 amino acids
(B) TYPE: amino acid
(C) STRANDEDNESS:
(D) TOPOLOGY: linear

(ii) MOLECULE TYPE: peptide
(v) FRAGMENT TYPE: internal
(xi) SEQUENCE DESCRIPTION: SEQ ID NO: 37:

```
Gln Leu Glu Ala Val Phe Glu Ala Asn Gln Asn Thr Lys Thr Ala Lys
1               5               10              15

Ile Glu Ile Lys Ala Ser Ile Asp Glu
        20              25
```

(2) INFORMATION FOR SEQ ID NO: 38:

(i) SEQUENCE CHARACTERISTICS:

    (A) LENGTH: 27 amino acids
    (B) TYPE: amino acid
    (C) STRANDEDNESS:
    (D) TOPOLOGY: linear

(ii) MOLECULE TYPE: peptide
(v) FRAGMENT TYPE: internal
(xi) SEQUENCE DESCRIPTION: SEQ ID NO: 38:

```
Asp Lys Gln Leu Glu Ala Val Phe Glu Ala Asn Gln Asn Thr Lys Thr
1               5                   10              15

Ala Lys Ile Glu Ile Lys Ala Ser Ile Asp Glu
            20                  25
```

(2) INFORMATION FOR SEQ ID NO: 39:

(i) SEQUENCE CHARACTERISTICS:

    (A) LENGTH: 28 amino acids
    (B) TYPE: amino acid
    (C) STRANDEDNESS:
    (D) TOPOLOGY: linear

(ii) MOLECULE TYPE: peptide
(v) FRAGMENT TYPE: internal
(xi) SEQUENCE DESCRIPTION: SEQ ID NO: 39:

```
Asp Lys Glu Gln Leu Glu Ala Val Phe Glu Ala Asn Gln Asn Thr Lys
1               5                   10              15

Thr Ala Lys Ile Glu Ile Lys Ala Ser Ile Asp Glu
            20                  25
```

(2) INFORMATION FOR SEQ ID NO: 40:

(i) SEQUENCE CHARACTERISTICS:

    (A) LENGTH: 29 amino acids
    (B) TYPE: amino acid
    (C) STRANDEDNESS:
    (D) TOPOLOGY: linear

(ii) MOLECULE TYPE: peptide
(v) FRAGMENT TYPE: internal
(xi) SEQUENCE DESCRIPTION: SEQ ID NO: 40:

```
Asp Lys Glu Gln Leu Glu Ala Val Phe Glu Ala Asn Gln Asn Thr Lys
1               5                   10              15

Thr Ala Lys Ile Glu Ile Lys Ala Ser Ile Asp Glu Glu
            20                  25
```

(2) INFORMATION FOR SEQ ID NO: 41:

    (i) SEQUENCE CHARACTERISTICS:

        (A) LENGTH: 27 amino acids
        (B) TYPE: amino acid
        (C) STRANDEDNESS:
        (D) TOPOLOGY: linear

    (ii) MOLECULE TYPE: peptide
    (v) FRAGMENT TYPE: internal
    (xi) SEQUENCE DESCRIPTION: SEQ ID NO: 41:

```
Asp Lys Gln Leu Glu Ala Val Phe Glu Ala Asn Gln Ala Thr Lys Thr
1               5               10                  15

Ala Lys Ile Glu Ile Lys Ala Ser Ile Asp Glu
            20              25
```

(2) INFORMATION FOR SEQ ID NO: 42:

    (i) SEQUENCE CHARACTERISTICS:

        (A) LENGTH: 25 amino acids
        (B) TYPE: amino acid
        (C) STRANDEDNESS:
        (D) TOPOLOGY: linear

    (ii) MOLECULE TYPE: peptide
    (v) FRAGMENT TYPE: internal
    (xi) SEQUENCE DESCRIPTION: SEQ ID NO: 42:

```
Asp Glu Lys Leu Glu Ala Val Phe Glu Ala Asn Gln Asn Thr Lys Thr
1               5               10                  15

Ala Lys Ile Glu Ile Lys Ala Ser Asp
            20              25
```

(2) INFORMATION FOR SEQ ID NO: 43:

    (i) SEQUENCE CHARACTERISTICS:

        (A) LENGTH: 24 amino acids
        (B) TYPE: amino acid
        (C) STRANDEDNESS:
        (D) TOPOLOGY: linear

    (ii) MOLECULE TYPE: peptide
    (v) FRAGMENT TYPE: internal
    (xi) SEQUENCE DESCRIPTION: SEQ ID NO: 43:

```
        Asp Lys Glu Leu Glu Ala Val Phe Glu Ala Asn Gln Asn Thr Lys Thr
        1               5                   10                  15

        Ala Lys Ile Glu Ile Lys Ala Asp
                    20
```

(2) INFORMATION FOR SEQ ID NO: 44:

    (i) SEQUENCE CHARACTERISTICS:

        (A) LENGTH: 24 amino acids
        (B) TYPE: amino acid
        (C) STRANDEDNESS:
        (D) TOPOLOGY: linear

    (ii) MOLECULE TYPE: peptide
    (v) FRAGMENT TYPE: internal
    (xi) SEQUENCE DESCRIPTION: SEQ ID NO: 44:

```
        Asp Lys Glu Leu Glu Ala Val Phe Glu Ala Asn Gln Asn Thr Lys Thr
        1               5                   10                  15

        Ala Lys Ile Glu Ile Lys Ala Lys
                    20
```

(2) INFORMATION FOR SEQ ID NO: 45:

    (i) SEQUENCE CHARACTERISTICS:

        (A) LENGTH: 23 amino acids
        (B) TYPE: amino acid
        (C) STRANDEDNESS:
        (D) TOPOLOGY: linear

    (ii) MOLECULE TYPE: peptide
    (v) FRAGMENT TYPE: internal
    (xi) SEQUENCE DESCRIPTION: SEQ ID NO: 45:

```
        Asp Lys Glu Leu Glu Ala Val Phe Glu Ala Asn Gln Asn Thr Lys Thr
        1               5                   10                  15

        Ala Lys Ile Glu Ile Lys Asp
                    20
```

(2) INFORMATION FOR SEQ ID NO: 46:

    (i) SEQUENCE CHARACTERISTICS:

        (A) LENGTH: 23 amino acids
        (B) TYPE: amino acid
        (C) STRANDEDNESS:
        (D) TOPOLOGY: linear

    (ii) MOLECULE TYPE: peptide
    (v) FRAGMENT TYPE: internal

(xi) SEQUENCE DESCRIPTION: SEQ ID NO: 46:

```
Asp Lys Glu Leu Glu Ala Val Phe Glu Ala Asn Gln Asn Thr Lys Thr
1               5               10              15

Ala Lys Ile Glu Ile Lys Lys
        20
```

(2) INFORMATION FOR SEQ ID NO: 47:

    (i) SEQUENCE CHARACTERISTICS:

        (A) LENGTH: 22 amino acids
        (B) TYPE: amino acid
        (C) STRANDEDNESS:
        (D) TOPOLOGY: linear

    (ii) MOLECULE TYPE: peptide
    (v) FRAGMENT TYPE: internal
    (xi) SEQUENCE DESCRIPTION: SEQ ID NO: 47:

```
Asp Lys Glu Leu Glu Ala Val Phe Glu Ala Asn Gln Asn Thr Lys Thr
1               5               10              15

Ala Lys Ile Glu Ile Lys
        20
```

(2) INFORMATION FOR SEQ ID NO: 48:

    (i) SEQUENCE CHARACTERISTICS:

        (A) LENGTH: 21 amino acids
        (B) TYPE: amino acid
        (C) STRANDEDNESS:
        (D) TOPOLOGY: linear

    (ii) MOLECULE TYPE: peptide
    (v) FRAGMENT TYPE: internal
    (xi) SEQUENCE DESCRIPTION: SEQ ID NO: 48:

```
Asp Lys Glu Leu Glu Ala Val Phe Glu Ala Asn Gln Asn Thr Lys Thr
1               5               10              15

Ala Lys Ile Glu Asp
        20
```

(2) INFORMATION FOR SEQ ID NO: 49:

    (i) SEQUENCE CHARACTERISTICS:

        (A) LENGTH: 21 amino acids
        (B) TYPE: amino acid
        (C) STRANDEDNESS:
        (D) TOPOLOGY: linear

(ii) MOLECULE TYPE: peptide
(v) FRAGMENT TYPE: internal
(xi) SEQUENCE DESCRIPTION: SEQ ID NO: 49:

```
Asp Lys Glu Leu Glu Ala Val Phe Glu Ala Asn Gln Asn Thr Lys Thr
1               5                   10                  15

Ala Lys Ile Glu Lys
            20
```

(2) INFORMATION FOR SEQ ID NO: 50:

(i) SEQUENCE CHARACTERISTICS:

(A) LENGTH: 20 amino acids
(B) TYPE: amino acid
(C) STRANDEDNESS:
(D) TOPOLOGY: linear

(ii) MOLECULE TYPE: peptide
(v) FRAGMENT TYPE: internal
(xi) SEQUENCE DESCRIPTION: SEQ ID NO: 50:

```
Asp Lys Glu Leu Glu Ala Val Phe Glu Ala Asn Gln Asn Thr Lys Thr
1               5                   10                  15

Ala Lys Ile Glu
            20
```

(2) INFORMATION FOR SEQ ID NO: 51:

(i) SEQUENCE CHARACTERISTICS:

(A) LENGTH: 15 amino acids
(B) TYPE: amino acid
(C) STRANDEDNESS:
(D) TOPOLOGY: linear

(ii) MOLECULE TYPE: peptide
(v) FRAGMENT TYPE: internal
(xi) SEQUENCE DESCRIPTION: SEQ ID NO: 51:

```
Leu Glu Ala Val Phe Glu Ala Asn Gln Asn Thr Lys Thr Ala Lys
1               5                   10                  15
```

(2) INFORMATION FOR SEQ ID NO: 52:

(i) SEQUENCE CHARACTERISTICS:

(A) LENGTH: 15 amino acids
(B) TYPE: amino acid
(C) STRANDEDNESS:
(D) TOPOLOGY: linear

(ii) MOLECULE TYPE: peptide

(v) FRAGMENT TYPE: internal
(xi) SEQUENCE DESCRIPTION: SEQ ID NO: 52:

```
Leu Glu Ala Val Phe Glu Ala Asn Gln Ala Thr Lys Thr Ala Lys
1               5                   10                  15
```

(2) INFORMATION FOR SEQ ID NO: 53:

(i) SEQUENCE CHARACTERISTICS:

(A) LENGTH: 16 amino acids
(B) TYPE: amino acid
(C) STRANDEDNESS:
(D) TOPOLOGY: linear

(ii) MOLECULE TYPE: peptide
(v) FRAGMENT TYPE: internal
(xi) SEQUENCE DESCRIPTION: SEQ ID NO: 53:

```
Asp Lys Thr Ala Lys Ile Glu Ile Lys Ala Ser Ile Asp Gly Leu Glu
1               5                   10                  15
```

(2) INFORMATION FOR SEQ ID NO: 54:

(i) SEQUENCE CHARACTERISTICS:

(A) LENGTH: 15 amino acids
(B) TYPE: amino acid
(C) STRANDEDNESS:
(D) TOPOLOGY: linear

(ii) MOLECULE TYPE: peptide
(v) FRAGMENT TYPE: internal
(xi) SEQUENCE DESCRIPTION: SEQ ID NO: 54:

```
Lys Thr Ala Lys Ile Glu Ile Lys Ala Ser Ile Asp Gly Leu Glu
1               5                   10                  15
```